(19) Europäisches Patentamt
European Patent Office
Office européen des brevets

(11) EP 3 086 760 B1

(12) **EUROPEAN PATENT SPECIFICATION**

(45) Date of publication and mention of the grant of the patent: **30.01.2019 Bulletin 2019/05**

(21) Application number: **14816277.9**

(22) Date of filing: **19.12.2014**

(51) Int Cl.:
*A61K 8/34* (2006.01)
*A61K 8/89* (2006.01)
*A61K 8/31* (2006.01)
*A61K 8/81* (2006.01)
*A61Q 1/06* (2006.01)
*A61K 8/02* (2006.01)
*A61K 8/891* (2006.01)

(86) International application number:
**PCT/EP2014/078901**

(87) International publication number:
**WO 2015/097110 (02.07.2015 Gazette 2015/26)**

(54) **SOLID COMPOSITION COMPRISING A VINYL POLYMER BEARING A CARBOSILOXANE DENDRIMER UNIT, VOLATILE HYDROCARBON-BASED OILS AND SOLID FATTY ALCOHOL, AND TREATMENT PROCESS**

FESTE ZUSAMMENSETZUNG ENTHALTEND EIN VINYLPOLYMER MIT EINER CARBOSILOXAN-DENDRIMER-EINHEIT, AUF BASIS FLÜCHTIGER KOHLENWASSERSTOFFÖLEN UND EINES FESTES ALKOHOLS SOWIE BEHANDLUNGSVERFAHREN

COMPOSITION SOLIDE COMPRENANT UN POLYMÈRE DE VINYLE PORTANT UNE UNITÉ DENDRIMÈRE DE CARBOSILOXANE, DES HUILES VOLATILES À BASE D’HYDROCARBURE ET UN ALCOOL GRAS SOLIDE, ET PROCÉDÉ DE TRAITEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2013 FR 1363369**
**23.12.2013 FR 1363370**
**23.12.2013 FR 1363368**

(43) Date of publication of application:
**02.11.2016 Bulletin 2016/44**

(73) Proprietor: **L’Oréal**
**75008 Paris (FR)**

(72) Inventors:
- **GUILLARD, Sylvie**
**F-77173 Chevry Cossigny (FR)**
- **MANET, Sylvie**
**F-91370 Verrières le Buisson (FR)**
- **OHANIAN, Cécile**
**75014 Paris (FR)**
- **ARDITTY, Stéphane**
**94550 Chevilly-la-Rue (FR)**
- **DEBEAUD, Roshanak**
**F-92240 L’hay les Roses (FR)**
- **PRUD‘HOMME, Estelle**
**94550 Chevilly-la-Rue (FR)**

(74) Representative: **Wattremez, Catherine**
**L’Oréal**
**Service DIPI**
**9 Rue Pierre Dreyfus**
**92110 Clichy (FR)**

(56) References cited:
**DE-A1-102008 022 434**
**FR-A1- 2 878 738**
**FR-A1- 2 968 979**
**US-A1- 2012 095 113**
**US-A1- 2012 269 875**

- **STÉPHANIE POSTIAUX KATRIN STEINBACH ESTERINA SCHIROSI ET AL: "Long lasting and meal resistance in lipsticks and lipcreams using silicone acrylate copolymers", RESEARCH DISCLOSURE, MASON PUBLICATIONS, HAMPSHIRE, GB, vol. 505, no. 25, 1 May 2006 (2006-05-01), XP007136165, ISSN: 0374-4353**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DOW CORNING SA ET AL: "The use of silicone acrylate copolymers in personal care", RESEARCH DISCLOSURE, MASON PUBLICATIONS, HAMPSHIRE, GB, vol. 485, no. 14, 1 September 2004 (2004-09-01), XP007134249, ISSN: 0374-4353**
- **STÉPHANIE POSTIAUX GIADA TONET ET AL: "Silicone acrylate copolymers in lipstick and color cosmetics", RESEARCH DISCLOSURE, MASON PUBLICATIONS, HAMPSHIRE, GB, vol. 495, no. 5, 1 July 2005 (2005-07-01), XP007135235, ISSN: 0374-4353**

## Description

The present invention relates to a composition in the form of an anhydrous solid cosmetic composition comprising a particular film-forming polymer and at least two hydrocarbon-based volatile oils, and also to a lip makeup and/or care process using the same.

Solid cosmetic compositions for making up and/or caring for the skin and/or the lips must satisfy several conditions regarding their mechanical characteristics and, quite obviously, regarding their performance qualities at the time of application of the composition, and also their evolution over time, once the composition is in place.

Thus, solid care and/or makeup compositions generally contain one or more fatty substances structured with a "structuring" or "gelling" agent, conventionally a wax or a polymer, to improve the stiffness of the compositions and especially to obtain solid compositions, preferably in the form of wands, which remain stable.

Needless to say, the galenical form of these compositions must, on the one hand, satisfy mechanical requirements in order to ensure the glidance and persistence of the stick during application and to prevent it from breaking, and, on the other hand, satisfy transfer qualities so as to ensure comfortable application and also a sufficient and good-quality deposit on the lips.

Furthermore, the deposited film must be sufficiently fine, non-tacky, and must not be too greasy or, on the other hand, give rise to any sensation of tautness. Finally, it should have a suitable level of gloss and have good persistence.

It is known practice to use, in cosmetic compositions, vinyl polymers containing at least one carbosiloxane dendrimer-based unit, whose presence improves the persistence of the deposited film. The starting material is available in the form of a mixture in a silicone or hydrocarbon-based volatile oil.

The solid anhydrous compositions prepared comprising this film-forming polymer usually contain a silicone volatile oil, a hydrocarbon-based volatile oil or a mixture of these two oils.

Document "Long lasting and meal resistance in lipsticks and lipcreams using silicone acrylate copolymers" (RESEARCH DISCLOSURE, MASON PUBLICATIONS, HAMPSHIRE, GB, vol. 505, no. 25, 1 May 2006) discloses a lipstick (liptissime duo stick) comprising such a dendrimer. It shows an improved long-lasting and is comfortable, easy to apply and is shiny. Document "Silicone acrylate copolymers in lipstick and color cosmetics" (RESEARCH DISCLOSURE, MASON PUBLICATIONS, HAMPSHIRE, GB, vol. 495, no. 5, 1 July 2005) discloses a non-transfer and long-lasting solid lipstick, which is also comfortable, based on the same dendrimer.

However, it has been found that, on application, the glidance of the composition and the comfort of the deposit thus obtained could be further improved. The aim of the present invention is thus to overcome the drawbacks described above.

These objectives are achieved by the present invention, one subject of which is thus a solid anhydrous cosmetic composition comprising:

a) at least one vinyl polymer comprising at least one carbosiloxane dendrimer-based unit;
b) at least two hydrocarbon-based volatile oils,
c) at least one saturated or unsaturated, preferably linear, solid fatty alcohol whose melting point is greater than or equal to 40°C, comprising from 16 to 60 carbon atoms, representing from 5% to 20% by weight, relative to the total weight of the composition.

The invention also relates to a process for making up and/or caring for the lips, in which the abovementioned composition is applied to the lips.

The composition according to the invention has the advantage of being strong enough to be able to be presented especially in the form of a lipstick wand, while at the same time being very easy to apply and glidant. It delivers a fine, homogeneous, non-greasy and non-tacky film of composition, which remains comfortable after application, and whose colour is homogeneous. The deposit also shows good persistence as well as an improved resistance to migration.

The present invention will emerge more clearly on reading the description and the examples that follow.

It should be noted that, in the remainder of the description, unless otherwise indicated, the limits indicated for a range are included in said range.

The expressions "at least one" and "several" are used without distinction.

The term "anhydrous" especially means that water is preferably not deliberately added to the compositions, but may be present in trace amounts in the various compounds used in the compositions.

The temperatures indicated later are indicated at atmospheric pressure ($1.013 \times 10^5$ Pa).

The cosmetic composition according to the invention is a composition more particularly intended for making up and/or caring for the skin or the lips. Preferably, the composition according to the invention is intended for making up and/or caring for the lips, and even more preferentially for making up the lips.

As indicated previously, the composition according to the invention is in a solid form. In particular, it is in the form of a stick (wand, pen) or in a form cast in a dish or in a jar.

Preferably, it is a lip balm and/or a lipstick, which is even more preferentially in the form of a stick (wand).
The hardness of the composition is measured according to the following protocol:

**Protocol for measuring the hardness**

The lipstick is stored at 20°C for 24 hours before measuring the hardness.
The hardness may be measured at 20°C via the "cheese wire" method, which consists in transversely cutting a wand of product, which is preferably a circular cylinder, by means of a rigid tungsten wire 250 μm in diameter, by moving the wire relative to the stick at a speed of 100 mm/minute.
The hardness of the samples of compositions of the invention, expressed in $Nm^{-1}$, is measured using a DFGS2 tensile testing machine from the company Indelco-Chatillon.
The measurement is repeated three times and then averaged. The average of the three values read using the tensile testing machine mentioned above, noted Y, is given in grams. This average is converted into newtons and then divided by L which represents the longest distance through which the wire passes. In the case of a cylindrical wand, L is equal to the diameter (in metres).
The hardness is converted into $Nm^{-1}$ by the equation below:

$$(Y \text{ x } 10^{-3} \text{ x } 9.8)/L$$

For a measurement at a different temperature, the stick is stored for 24 hours at this new temperature before the measurement.
According to this measuring method, the composition according to the invention advantageously has a hardness at 20°C and at atmospheric pressure of greater than or equal to 40 $Nm^{-1}$. According to one particular mode, the hardness at 20°C and at atmospheric pressure is greater than or equal to 60 $Nm^{-1}$.
Preferably, the composition according to the invention has a hardness at 20°C of less than 500 $Nm^{-1}$, especially less than 400 $Nm^{-1}$ and preferably less than 300 $Nm.^{-1}$.
Preferably, the composition has a hardness of between 60 and 150 $Nm^{-1}$.

**VINYL POLYMER/CARBOSILOXANE DENDRIMER UNIT(S)**

As indicated previously, the composition according to the invention comprises at least one vinyl polymer comprising at least one carbosiloxane dendrimer-based unit.
The vinyl polymer(s) have a backbone and at least one side chain, which comprises a carbosiloxane dendrimer-based unit having a carbosiloxane dendrimer structure.
The term "carbosiloxane dendrimer structure" in the context of the present invention represents a molecular structure with branched groups of high molecular weights, said structure having high regularity in the radial direction starting from the bond to the backbone. Such carbosiloxane dendrimer structures are described in the form of a highly branched siloxane-silylalkylene copolymer in Japanese patent application JP 9-171 154.
A vinyl polymer according to the invention may contain carbosiloxane dendrimer-based units that may be represented by the following general formula (I):

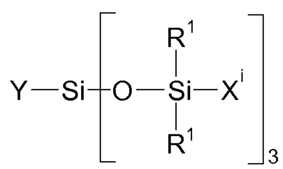

(I)

in which:

- $R^1$ represents an aryl group containing from 5 to 10 carbon atoms or an alkyl group containing from 1 to 10 carbon atoms;
- $X^i$ represents a silylalkyl group which, when i = 1, is represented by formula (II):

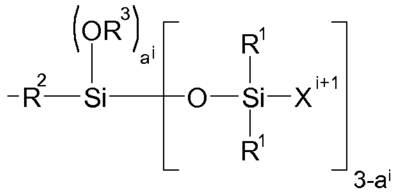

(II)

in which:

- $R^1$ is as defined above in formula (I),
- $R^2$ represents an alkylene radical containing from 2 to 10 carbon atoms,
- $R^3$ represents an alkyl group containing from 1 to 10 carbon atoms,
- $X^{i+i}$ is chosen from: a hydrogen atom, an alkyl group containing from 1 to 10 carbon atoms, an aryl group containing from 5 to 10 carbon atoms and a silylalkyl group defined above of formula (II) with i = i + 1,

- i is an integer from 1 to 10 which represents the generation of said silylalkyl group, and
- $a^i$ is an integer from 0 to 3;

- Y represents a radical-polymerizable organic group chosen from:

- organic groups containing a methacrylic group or an acrylic group, said organic groups being represented by the formulae:

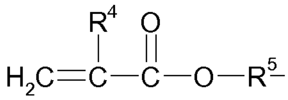

or

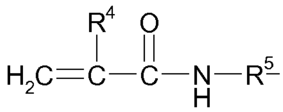

in which:

* $R^4$ represents a hydrogen atom or an alkyl group containing from 1 to 10 carbon atoms; and
* $R^5$ represents an alkylene group containing from 1 to 10 carbon atoms, such as a methylene group, an ethylene group, a propylene group or a butylene group, methylene and propylene groups being preferred; and

- organic groups containing a styryl group of formula:

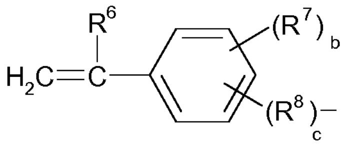

in which:

* $R^6$ represents a hydrogen atom or an alkyl group containing from 1 to 10 carbon atoms, such as a methyl group, an ethyl group, a propyl group or a butyl group, the methyl group being preferred;
* $R^7$ represents an alkyl group containing from 1 to 10 carbon atoms;
* $R^8$ represents an alkylene group containing from 1 to 10 carbon atoms, such as a methylene group, an ethylene group, a propylene group or a butylene group, the ethylene group being preferred;
* b is an integer from 0 to 4; and
* c is 0 or 1, such that, if c is 0, $-(R^8)_c-$ represents a bond.

According to one embodiment, $R^1$ may represent an aryl group containing from 5 to 10 carbon atoms or an alkyl group containing from 1 to 10 carbon atoms. The alkyl group may preferably be represented by a methyl group, an ethyl group, a propyl group, a butyl group, a pentyl group, an isopropyl group, an isobutyl group, a cyclopentyl group or a cyclohexyl group. The aryl group may preferably be represented by a phenyl group and a naphthyl group. The

methyl and phenyl groups are more particularly preferred, and the methyl group is preferred among all.

According to one embodiment, $R^2$ represents an alkylene group containing from 2 to 10 carbon atoms, in particular a linear alkylene group, such as an ethylene, propylene, butylene or hexylene group; or a branched alkylene group, such as a methylmethylene, methylethylene, 1-methylpentylene or 1,4-dimethylbutylene group.

The ethylene, methylethylene, hexylene, 1-methylpentylene and 1,4-dimethylbutylene groups are preferred among all.

According to one embodiment, $R^3$ is chosen from methyl, ethyl, propyl, butyl and isopropyl groups.

In formula (II), i indicates the number of generations and thus corresponds to the number of repeats of the silylalkyl group.

For example, when the generation number is equal to 1, the carbosiloxane dendrimer may be represented by the general formula shown below, in which Y, $R^1$, $R^2$ and $R^3$ are as defined above, $R^{12}$ represents a hydrogen atom or is identical to $R^1$; $a^1$ is identical to $a^i$. Preferably, the total average number of groups $OR^3$ in a molecule is within the range from 0 to 7.

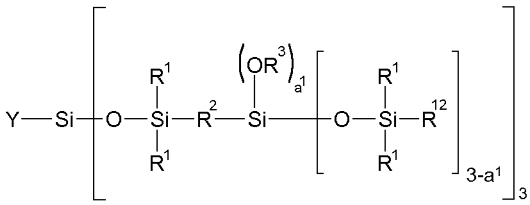

When the generation number is equal to 2, the carbosiloxane dendrimer may be represented by the general formula below, in which Y, $R^1$, $R^2$, $R^3$ and $R^{12}$ are the same as defined above; $a^1$ and $a^2$ represent the $a^i$ of the indicated generation. Preferably, the total average number of groups $OR^3$ in a molecule is within the range from 0 to 25.

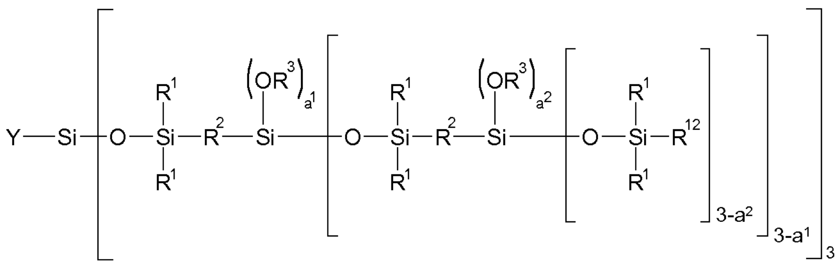

When the generation number is equal to 3, the carbosiloxane dendrimer is represented by the general formula below, in which Y, $R^1$, $R^2$, $R^3$ and $R^{12}$ are the same as defined above; $a^1$, $a^2$ and $a^3$ represent the $a^i$ of the indicated generation. Preferably, the total average number of groups $OR^3$ in a molecule is within the range from 0 to 79.

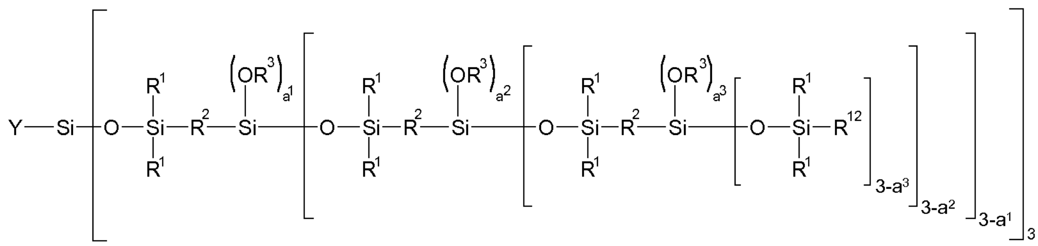

A vinyl polymer bearing at least one carbosiloxane dendrimer-based unit has a molecular side chain containing a carbosiloxane dendrimer structure, and may be derived from the polymerization of:

(A) from 0 to 99.9 parts by weight of a vinyl monomer; and
(B) from 100 to 0.1 parts by weight of a carbosiloxane dendrimer containing a radical-polymerizable organic group, represented by general formula (I) as defined above.

The monomer of vinyl type that is the component (A) in the vinyl polymer bearing at least one carbosiloxane dendrimer-based unit is a monomer of vinyl type that contains a radical-polymerizable vinyl group.

There is no particular limitation as regards such a monomer.

The following are examples of this monomer of vinyl type: methyl methacrylate, ethyl methacrylate, n-propyl methacrylate, isopropyl methacrylate or a methacrylate of an analogous lower alkyl; glycidyl methacrylate; butyl methacrylate, butyl acrylate, n-butyl methacrylate, isobutyl methacrylate, tert-butyl acrylate, tert-butyl methacrylate, n-hexyl methacrylate, cyclohexyl methacrylate, 2-ethylhexyl acrylate, 2-ethylhexyl methacrylate, octyl methacrylate, lauryl methacrylate, stearyl acrylate, stearyl methacrylate or a higher-analogue methacrylate; vinyl acetate, vinyl propionate or a vinyl ester of an analogous lower fatty acid; vinyl caproate, vinyl 2-ethylhexoate, vinyl laurate, vinyl stearate or an ester of a higher fatty acid analogue; styrene, vinyltoluene, benzyl methacrylate, phenoxyethyl methacrylate, vinylpyrrolidone or similar vinylaromatic monomers; methacrylamide, N-methylolmethacrylamide, N-methoxymethylmethacrylamide, isobutoxymethoxymethacrylamide, N,N-dimethylmethacrylamide or similar monomers of vinyl type containing amide groups; hydroxyethyl methacrylate, hydroxypropyl alcohol methacrylate or similar monomers of vinyl type containing hydroxyl groups; acrylic acid, methacrylic acid, itaconic acid, crotonic acid, fumaric acid, maleic acid or similar monomers of vinyl type containing a carboxylic acid group; tetrahydrofurfuryl methacrylate, butoxyethyl methacrylate, ethoxydiethylene glycol methacrylate, polyethylene glycol methacrylate, polypropylene glycol monomethacrylate, hydroxybutyl vinyl ether, cetyl vinyl ether, 2-ethylhexyl vinyl ether or a similar monomer of vinyl type with ether bonds; methacryloxypropyltrimethoxysilane, polydimethylsiloxane containing a methacrylic group on one of its molecular ends, polydimethylsiloxane containing a styryl group on one of its molecular ends, or a similar silicone compound containing unsaturated groups; butadiene; vinyl chloride; vinylidene chloride; methacrylonitrile; dibutyl fumarate; anhydrous maleic acid; anhydrous succinic acid; methacryl glycidyl ether; an organic salt of an amine, an ammonium salt, and an alkali metal salt of methacrylic acid, of itaconic acid, of crotonic acid, of maleic acid or of fumaric acid; a radical-polymerizable unsaturated monomer containing a sulfonic acid group such as a styrenesulfonic acid group; a quaternary ammonium salt derived from methacrylic acid, such as 2-hydroxy-3-methacryloxypropyltrimethylammonium chloride; and a methacrylic acid ester of an alcohol containing a tertiary amine group, such as a methacrylic acid ester of diethylamine.

Multifunctional monomers of vinyl type may also be used.

The following represent examples of such compounds: trimethylolpropane trimethacrylate, pentaerythrityl trimethacrylate, ethylene glycol dimethacrylate, tetraethylene glycol dimethacrylate, polyethylene glycol dimethacrylate, 1,4-butanediol dimethacrylate, 1,6-hexanediol dimethacrylate, neopentyl glycol dimethacrylate, trimethylolpropanetrioxyethyl methacrylate, tris(2-hydroxyethyl)isocyanurate dimethacrylate, tris(2-hydroxyethyl)isocyanurate trimethacrylate, polydimethylsiloxane capped with styryl groups containing divinylbenzene groups on both ends, or similar silicone compounds containing unsaturated groups.

A carbosiloxane dendrimer, which is the component (B), may be represented by formula (I) as defined above.

The following represent the preferred examples of group Y of formula (I): an acryloxymethyl group, a 3-acryloxypropyl group, a methacryloxymethyl group, a 3-methacryloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, a vinyl group, an allyl group, a methallyl group and a 5-hexenyl group.

A carbosiloxane dendrimer according to the present invention may be represented by the formulae having the average structures below:

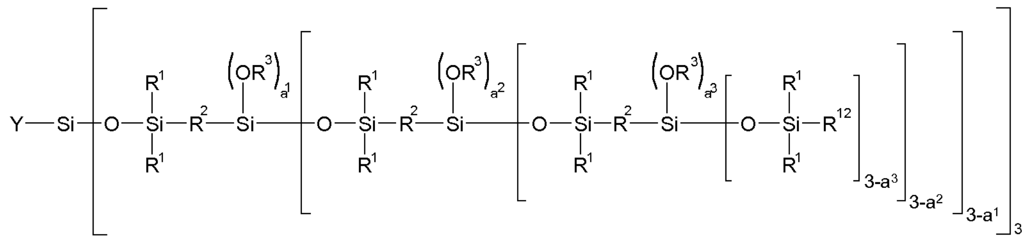

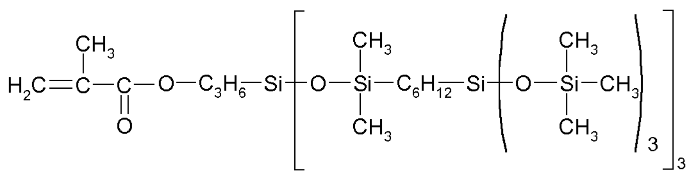

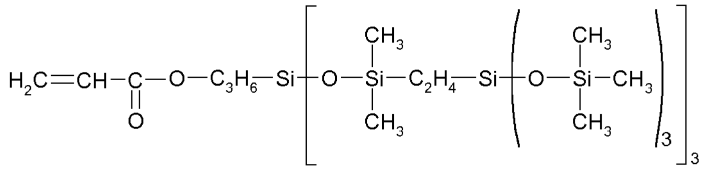

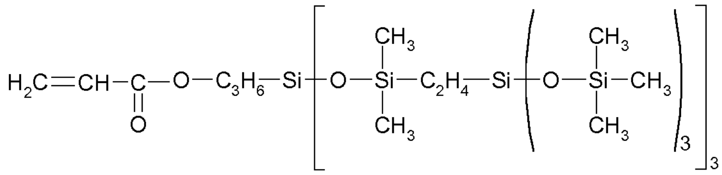

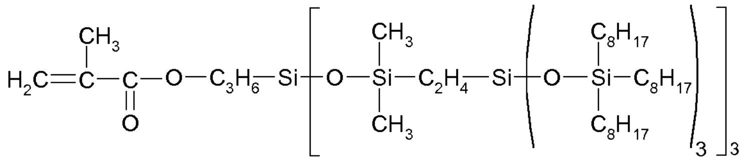

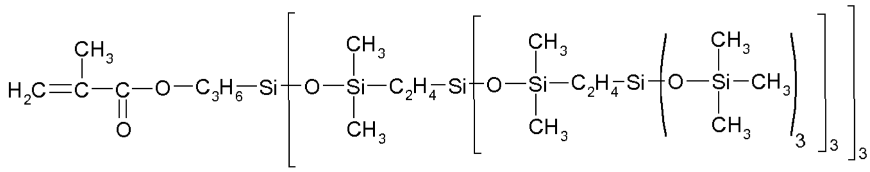

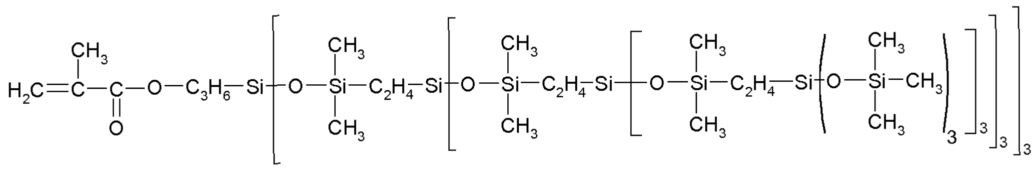

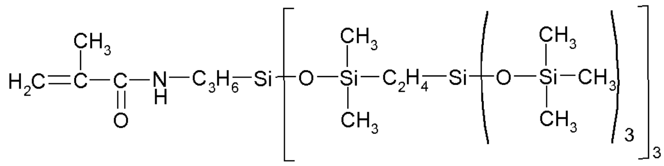

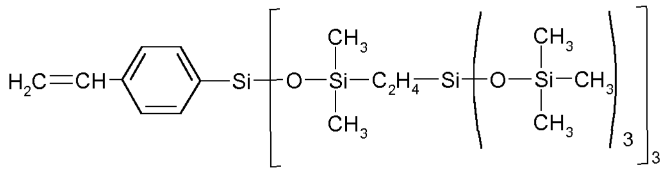

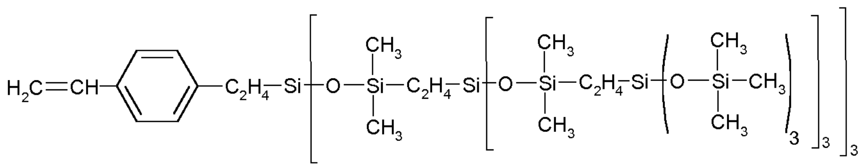

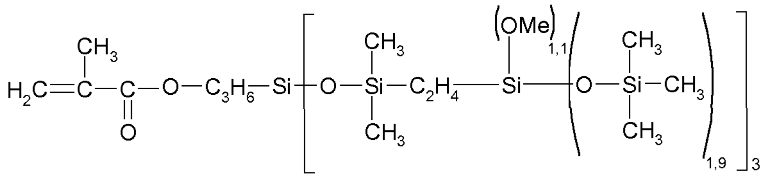

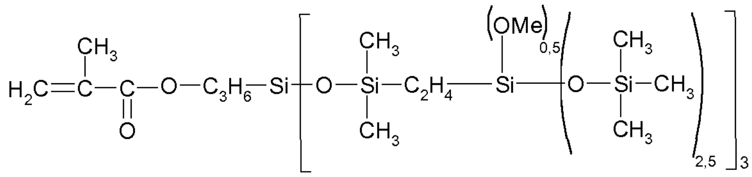

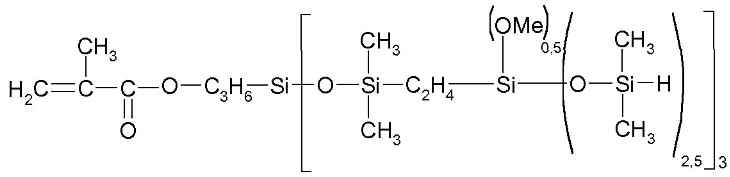

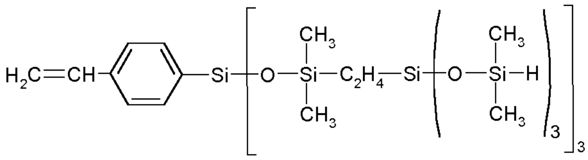

Thus, according to one embodiment, the carbosiloxane dendrimer of the composition according to the present invention is represented by the following formula:

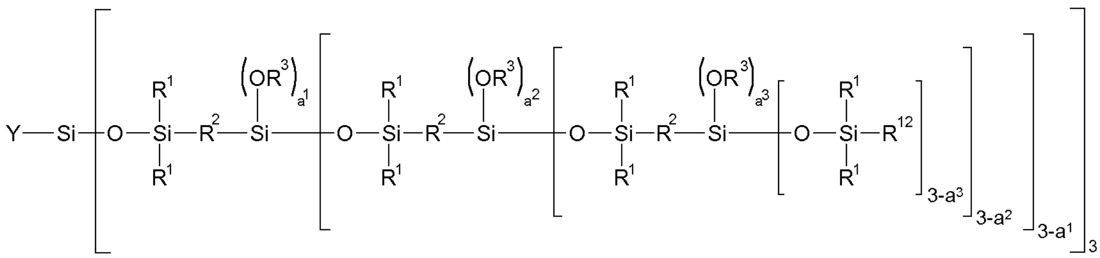

in which:

- Y, $R^1$, $R^2$ and $R^3$ are as defined in formulae (I) and (II) above;
- $a^1$, $a^2$ and $a^3$ correspond to the definition of $a^i$ according to formula (II); and
- $R^{12}$ is H, an aryl group containing from 5 to 10 carbon atoms or an alkyl group containing from 1 to 10 carbon atoms.

According to one embodiment, the carbosiloxane dendrimer of the composition according to the present invention is represented by one of the following formulae:

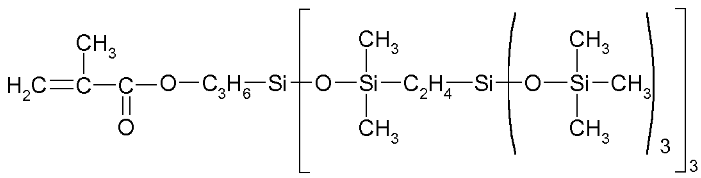

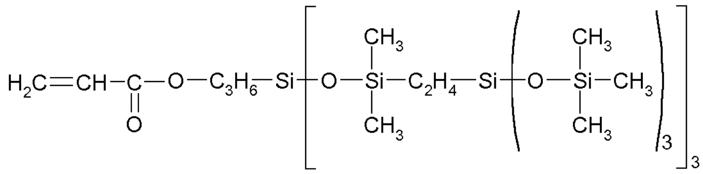

The vinyl polymer comprising the carbosiloxane dendrimer according to the invention may be manufactured according to the process for manufacturing a branched silalkylene siloxane described in Japanese patent application Hei 9-171 154.

For example, it may be produced by subjecting an organosilicon compound containing a hydrogen atom linked to a silicon atom, represented by the following general formula (IV):

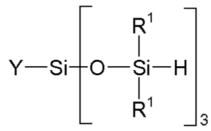
(IV)

$R^1$ being as defined above in formula (I),
and an organosilicon compound containing an alkenyl group, to a hydrosilylation reaction.

In the above formula, the organosilicon compound may be represented by 3-methacryloxypropyltris(dimethylsiloxy)silane, 3-acryloxypropyltris(dimethylsiloxy)silane and 4-vinylphenyltris(dimethylsiloxy)silane. The organosilicon compound that contains an alkenyl group may be represented by vinyltris(trimethylsiloxy)silane, vinyltris(dimethylphenylsiloxy)silane, and 5-hexenyltris(trimethylsiloxy)silane.

The hydrosilylation reaction is performed in the presence of a chloroplatinic acid, a complex of vinylsiloxane and of platinum, or a similar transition metal catalyst.

A vinyl polymer containing at least one carbosiloxane dendrimer-based unit may be chosen from polymers such that the carbosiloxane dendrimer-based unit is a carbosiloxane dendritic structure represented by formula (III):

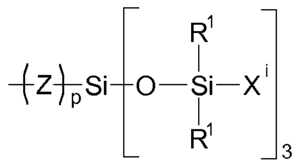
(III)

in which Z is a divalent organic group, "p" is 0 or 1, $R^1$ is as defined above in formula (IV) and X' is a silylalkyl group represented by formula (II) as defined above.

In a vinyl polymer bearing at least one carbosiloxane dendrimer-based unit, the polymerization ratio between the components (A) and (B), in terms of the weight ratio between (A) and (B), is within a range from 0/100 to 99.9/0.1, or even from 0.1/99.9 to 99.9/0.1 and preferably within a range from 1/99 to 99/1. A ratio between the components (A) and (B) of 0/100 means that the compound becomes a homopolymer of component (B).

A vinyl polymer bearing at least one carbosiloxane dendrimer-based unit may be obtained by copolymerization of the components (A) and (B), or by polymerization of the component (B) alone.

The polymerization may be a free-radical polymerization or an ionic polymerization, but free-radical polymerization is preferred.

The polymerization may be performed by bringing about a reaction between the components (A) and (B) in a solution for a period of from 3 to 20 hours in the presence of a radical initiator at a temperature of from 50°C to 150°C.

A suitable solvent for this purpose is hexane, octane, decane, cyclohexane or a similar aliphatic hydrocarbon; benzene, toluene, xylene or a similar aromatic hydrocarbon; diethyl ether, dibutyl ether, tetrahydrofuran, dioxane or ethers; acetone, methyl ethyl ketone, methyl isobutyl ketone, diisobutyl ketone or similar ketones; methyl acetate, ethyl acetate, butyl acetate, isobutyl acetate or similar esters; methanol, ethanol, isopropanol, butanol or similar alcohols; octamethylcyclotetrasiloxane, decamethylcyclopentasiloxane, hexamethyldisiloxane, octamethyltrisiloxane or a similar organosiloxane oligomer.

A radical initiator may be any compound known in the art for standard free-radical polymerization reactions.

The specific examples of such radical initiators are 2,2'-azobis(isobutyronitrile), 2,2'-azobis(2-methylbutyronitrile), 2,2'-azobis(2,4-dimethylvaleronitrile) or similar compounds of azobis type; benzoyl peroxide, lauroyl peroxide, tert-butyl peroxybenzoate, tert-butyl peroxy-2-ethylhexanoate or a similar organic peroxide. These radical initiators may be used alone or in a combination of two or more. The radical initiators may be used in an amount of from 0.1 to 5 parts by weight per 100 parts by weight of the components (A) and (B). A chain-transfer agent may be added. The chain-transfer agent may be 2-mercaptoethanol, butyl mercaptan, n-dodecyl mercaptan, 3-mercaptopropyltrimethoxysilane, a polydimethylsiloxane containing a mercaptopropyl group or a similar compound of mercapto type; methylene chloride, chloroform, carbon tetrachloride, butyl bromide, 3-chloropropyltrimethoxysilane or a similar halogenated compound.

In the manufacture of the polymer of vinyl type, after the polymerization, the unreacted residual vinyl monomer may be removed under conditions of heating under vacuum.

To facilitate the preparation of starting material for cosmetic products, the number-average molecular weight of the vinyl polymer bearing a carbosiloxane dendrimer may be chosen within the range between 3000 and 2 000 000 and preferably between 5000 and 800 000. It may be a liquid, a gum, a paste, a solid, a powder, or any other form. The preferred forms are solutions consisting of the dilution of a dispersion or of a powder in solvents.

The vinyl polymer may be a dispersion of a polymer of vinyl type bearing a carbosiloxane dendrimer structure in its side molecular chain, in a liquid such as a silicone oil, an organic oil, an alcohol or water.

The silicone oil may be a dimethylpolysiloxane having the two molecular ends capped with trimethylsiloxy groups, a copolymer of methylphenylsiloxane and of dimethylsiloxane having the two molecular ends capped with trimethylsiloxy groups, a copolymer of methyl-3,3,3-trifluoropropylsiloxane and of dimethylsiloxane having the two molecular ends capped with trimethylsiloxy groups, or similar unreactive linear silicone oils, and also hexamethylcyclotrisiloxane, octamethylcyclotetrasiloxane, decamethylcyclopentasiloxane, dodecamethylcyclohexasiloxane or a similar cyclic compound. In addition to the unreactive silicone oils, modified polysiloxanes containing functional groups such as silanol groups, amino groups and polyether groups on the ends or within the molecular side chains may be used.

The organic oils may be isododecane, liquid paraffin, isoparaffin, hexyl laurate, isopropyl myristate, myristyl myristate, cetyl myristate, 2-octyldodecyl myristate; isopropyl palmitate, 2-ethylhexyl palmitate, butyl stearate, decyl oleate, 2-octyldodecyl oleate, myristyl lactate, cetyl lactate, lanolin acetate, stearyl alcohol, cetostearyl alcohol, oleyl alcohol, avocado oil, almond oil, olive oil, cocoa oil, jojoba oil, gum oil, sunflower oil, soybean oil, camellia oil, squalane, castor oil, cottonseed oil, coconut oil, egg yolk oil, polypropylene glycol monooleate, neopentyl glycol 2-ethylhexanoate or a similar glycol ester oil; triglyceryl isostearate, the triglyceride of a fatty acid of coconut oil, or a similar oil of a polyhydric alcohol ester; polyoxyethylene lauryl ether, polyoxypropylene cetyl ether or a similar polyoxyalkylene ether.

The alcohol may be any type that is suitable for use in combination with a cosmetic product starting material. For example, it may be methanol, ethanol, butanol, isopropanol or similar lower alcohols.

A solution or a dispersion of the alcohol should have a viscosity within the range from 10 to $10^9$ mPa at 25°C. To improve the sensory use properties in a cosmetic product, the viscosity should be within the range from 100 to $5 \times 10^8$ mPa.s.

The solutions and dispersions may be readily prepared by mixing a vinyl polymer bearing at least one carbosiloxane dendrimer-based unit with a silicone oil, an organic oil, an alcohol or water. The liquids may be present in the polymerization step. In this case, the unreacted residual vinyl monomer should be completely removed by heat treatment of the solution or dispersion under atmospheric pressure or reduced pressure.

In the case of a dispersion, the dispersity of the polymer of vinyl type may be improved by adding a surfactant.

Such an agent may be hexylbenzenesulfonic acid, octylbenzenesulfonic acid, decylbenzenesulfonic acid, dodecylbenzenesulfonic acid, cetylbenzenesulfonic acid, myristylbenzenesulfonic acid or anionic surfactants of the sodium salts of these acids; octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow-trimethylammonium hydroxide, coconut oil-trimethylammonium hydroxide, or a similar cationic surfactant; a polyoxyalkylene alkyl ether, a polyoxyalkylenealkylphenol, a polyoxyalkylene alkyl ester, the sorbitol ester of polyoxyalkylene, polyethylene glycol, polypropylene glycol, an ethylene oxide additive of diethylene glycol trimethylnonanol, and nonionic surfactants of polyester type, and also mixtures.

In the dispersion, a mean particle diameter of the polymer of vinyl type may be within a range of between 0.001 and 100 microns and preferably between 0.01 and 50 microns. The reason for this is that, outside the recommended range, a cosmetic product mixed with the emulsion will not have a nice enough feel in particular on the lips or to the touch, nor sufficient spreading properties nor a pleasant feel.

A vinyl polymer contained in the dispersion or the solution may have a concentration within a range of between 0.1% and 95% by weight and preferably between 5% and 85% by weight. However, to facilitate the handling and the preparation of the mixture, the range should preferably be between 10% and 75% by weight.

A vinyl polymer that is suitable for use in the invention may also be one of the polymers described in the examples of patent application EP 0 963 751.

According to one preferred embodiment, a vinyl polymer grafted with a carbosiloxane dendrimer may be the

product of polymerization of:

(A1) from 0 to 99.9 parts by weight of one or more acrylate or methacrylate monomer(s); and
(B1) from 100 to 0.1 parts by weight of an acrylate or methacrylate monomer of a tris[tri(trimethylsiloxy)silylethyldimethylsiloxy]silylpropyl carbosiloxane dendrimer.

The monomers (A1) and (B1) correspond respectively to specific monomers (A) and (B).

According to one embodiment, a vinyl polymer containing at least one carbosiloxane dendrimer-based unit may comprise a tris[tri(trimethylsiloxy)silylethyldimethylsiloxy]silylpropyl carbosiloxane dendrimer-based unit corresponding to one of the formulae:

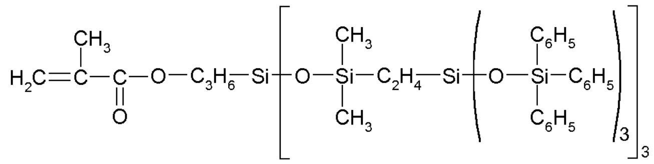

or

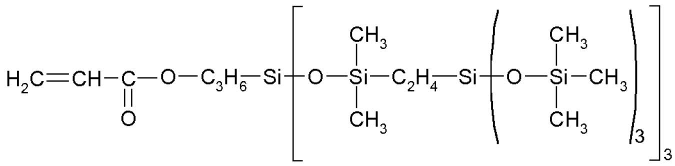

According to one preferred mode, a vinyl polymer bearing at least one carbosiloxane dendrimer-based unit used in the invention comprises at least one butyl acrylate monomer.

According to one embodiment, a vinyl polymer may also comprise at least one fluoro organic group.

Structures in which the polymerized vinyl units constitute the backbone and carbosiloxane dendritic structures and also fluoro organic groups are attached to side chains are particularly preferred.

The fluoro organic groups may be obtained by replacing with fluorine atoms all or some of the hydrogen atoms of methyl, ethyl, propyl, isopropyl, butyl, isobutyl, tert-butyl, pentyl, neopentyl, hexyl, cyclohexyl, heptyl, octyl, nonyl, decyl, undecyl, dodecyl, tridecyl, tetradecyl, hexadecyl and octadecyl groups and other alkyl groups of 1 to 20 carbon atoms, and also alkyloxyalkylene groups of 6 to 22 carbon atoms.

The groups represented by the formula $-(CH_2)_x-(CF_2)_y-R^{13}$ are suggested as examples of fluoroalkyl groups obtained by substituting fluorine atoms for hydrogen atoms of alkyl groups. In the formula, the index "x" is 0, 1, 2 or 3, and "y" is an integer from 1 to 20. $R^{13}$ is an atom or a group chosen from a hydrogen atom, a fluorine atom, $-CH(CF_3)_2-$ or $CF(CF_3)_2$. Such fluorine-substituted alkyl groups are exemplified by linear or branched polyfluoroalkyl or perfluoroalkyl groups represented by the formulae shown below:
$-CF_3$, $-C_2F_5$, $-nC_3F_7$, $-CF(CF_3)_2$, $-nC_4F_9$, $CF_2CF(CF_3)_2$, $-nC_5F_{11}$, $-nC_6F_{13}$,$-nC_8F_{17}$, $CH_2CF_3$, $-(CH(CF_3)_2$, $CH_2CH(CF_3)_2$-$CH_2(CF_2)_2F$, $-CH_2(CF_2)_3F$,$-CH_2(CF_2)_4F$,$CH_2(CF_2)_6F$, $CH_2(CF_2)_8F$, $-CH_2CH_2CF_3$, $-CH_2CH_2(CF_2)_2F$,$-CH_2CH_2(CF_2)_3F$, $-CH_2CH_2(CF_2)_4F$, $-CH_2CH_2(CF_2)_6F$, $-CH_2CH_2(CF_2)_8F$, $-CH_2CH_2(CF_2)_{10}F$, $-CH_2CH_2(CF_2)_{12}F$, $CH_2CH_2(CF_2)_{14}F$,$-CH_2CH_2(CF_2)_{16}F$, $-CH_2CH_2CH_2CF_3$, $-CH_2CH_2CH_2(CF_2)_2F$, $-CH_2CH_2CH_2(CF_2)_2H$, $-CH_2(CF_2)_4H$ and $-CH_2CH_2(CF_2)_3H$.

The groups represented by $-CH_2CH_2-(CF_2)_m-CFR^{14}-[OCF_2CF(CF_3)]_n-OC_3F_7$ are suggested as fluoroalkyloxyfluoroalkylene groups obtained by substituting fluorine atoms for hydrogen atoms of alkyloxyalkylene groups. In the formula, the index "m" is 0 or 1, "n" is 0, 1, 2, 3, 4 or 5, and $R^{14}$ is a fluorine atom or $CF_3$. Such fluoroalkyloxyfluoroalkylene groups are exemplified by the perfluoroalkyloxyfluoroalkylene groups represented by the formulae shown below:$-CH_2CH_2CF(CF_3)-[OCF_2CF(CF_3)]_n-OC_3F_7$, $-CH_2CH_2CF_2CF_2-[OCF_2CF(CF_3)]_n-OC_3F_7$.

The number-average molecular weight of the vinyl polymer used in the present invention may be between 3000 and 2 000 000 and more preferably between 5000 and 800 000.

This type of fluorinated vinyl polymer may be obtained by addition:

- of a vinyl monomer (M2) without a fluoro organic group,
- on a vinyl monomer (M1) containing fluoro organic groups, and
- a carbosiloxane dendrimer (B) as defined above, of general formula (I) as defined above,

by subjecting them to a copolymerization.

Thus, according to one embodiment, a composition of the invention may comprise a vinyl polymer bearing at least one carbosiloxane dendrimer-based unit and which results from the copolymerization of a vinyl monomer (M1) as defined above, optionally of a vinyl monomer (M2) as defined above, and of a carbosiloxane dendrimer (B) as defined above, said vinyl polymer having a copolymerization ratio between the monomer (M1) and the monomer (M2) of 0.1 to 100:99.9 to 0% by weight, and a copolymerization ratio between the sum of the monomers (M1) and (M2) and the monomer (B) of 0.1 to 99.9:99.9 to 0.1% by weight.

The vinyl monomers (M1) containing fluoro organic groups in the molecule are preferably monomers represented by the general formula:

$$(CH^2)=CR^{15}COOR^f.$$

In this formula, $R^{15}$ is a hydrogen atom or a methyl group and $R^f$ is a fluoro organic group exemplified by the fluoroalkyl and fluoroalkyloxyfluoroalkylene groups described above. The compounds represented by the formulae presented below are suggested as specific examples of the component (M1). In the formulae presented below, "z" is an integer from 1 to 4.

$CH_2=CCH_3COO-CF_3$, $CH_2=CCH_3COO-C_2F_5$, $CH_2=CCH3COO-nC_3F_7$,
$CH_2=CCH_3COO-CF(CF_3)_2$, $CH_2=CCH_3COO-nC_4F_9$,
$CH_2=CCH_3COO-CF(CF_3)_2$, $CH_2=CCH_3COO-nC_5F_{11}$,
$CH_2=CCH_3COO-nC_6F_{13}$, $CH_2=CCH_3COO-nC_8F_{17}$, $CH_2=CCH_3COO-CH_2CF_3$,
$CH_2=CCH_3COO-CH(CF_3)_2$, $CH_2=CCH_3COO-CH_2CH(CF_3)_2$,
$CH_2=CCH_3COO-CH_2(CF_2)_2F$, $CH_2=CCH_3COO-CH_2(CF_2)_2F$,
$CH_2=CCH_3COO-CH_2(CF_2)_4F$, $CH_2=CCH_3COO-CH_2(CF_2)_6F$,
$CH_2=CCH_3COO-CH_2(CF_2)_8F$, $CH_2=CCH_3COO-CH_2CH_2CF_3$,
$CH_2=CCH_3COO-CH_2CH_2(CF_2)_2F$, $CH_2=CCH_3COO-CH_2CH_2(CF_2)_3F$,
$CH_2=CCH_3COO-CH_2CH_2(CF_2)_4F$, $CH_2=CCH_3COO-CH_2CH_2(CF_2)_6F$,
$CH_2=CCH_3COO-CH_2CH_2(CF_2)_8F$, $CH_2=CCH_3COO-CH_2CH_2(CF_2)_{10}F$,
$CH_2=CCH_3COO-CH_2CH_2(CF_2)_{12}F$, $CH_2=CCH_3COO-CH_2CH_2(CF_2)_{14}F$,
$CH_2=CCH_3COO-CH_2-CH_2-(CF_2)_{16}F$, $CH_2=CCH_3COO-CH_2CH_2CH_2CF_3$,
$CH_2=CCH_3COO-CH_2CH_2CH_2(CF_2)_2F$, $CH_2=CCH_3COO-CH_2CH_2CH_2(CF_2)_2H$,
$CH_2=CCH_3COO-CH_2(CF_2)_4H$, $CH_2=CCH_3COO-(CF_2)_3H$,
$CH_2=CCH_3COO-CH_2CH_2CF(CF_3)-[OCF_2-CF(CF_3)]z-OC_3F_7$,
$CH_2=CCH_3COO-CH_2CH_2CF_2CF_2-[OCF_2-CF(CF_3)]z-OC_3F_7$,
$CH_2=CHCOO-CF_3$, $CH_2=CHCOO-C_2F_5$, $CH_2=CHCOO-nC_3F_7$,
$CH_2=CHCOO-CF(CF_3)_2$, $CH_2=CHCOO-nC_4F_9$, $CH_2=CHCOO-CF_2CF(CF_3)_2$,
$CH_2=CHCOO-nC_5F_{11}$, $CH_2=CHCOO-nC_6F_{13}$, $CH_2=CHCOO-nC_8F_{17}$,
$CH_2=CHCOO-CH_2CF_3$, $CH_2=CHCOO-CH(CF_3)_2$, $CH_2=CHCOO-CH_2CH(CF_3)_2$,
$CH_2=CHCOO-CH_2(CF_2)_2F$, $CH_2=CHCOO-CH_2(CF_2)_3F$,
$CH_2=CHCOO-CH_2(CF_2)_4F$, $CH_2=CHCOO-CH_2(CF_2)_6F$,
$CH_2=CHCOO-CH_2(CF_2)_8F$, $CH_2=CHCOO-CH_2CH_2CF_3$,
$CH_2=CHCOO-CH_2CH_2(CF_2)_2F$, $CH_2=CHCOO-CH_2CH_2(CF_2)_3F$,
$CH_2=CHCOO-CH_2CH_2(CF_2)_4F$, $CH_2=CHCOO-CH_2CH_2(CF_2)_6F$,
$CH_2=CHCOO-CH_2CH_2(CF_2)_8F$, $CH_2=HCOO-CH_2CH_2(CF_2)_{10}F$,
$CH_2-CHCOO-CH_2CH_2-(CF_2)_{12}F$, $CH_2=CHCOO-CH_2CH_2(CF_2)_{14}F$,
$CH_2=CHCOO-CH_2CH_2(CF_2)_{16}F$, $CH_2=CHCOO-CH_2CH_2CH_2CF_3$,
$CH_2=CHCOO-CH_2CH_2CH_2(CF_2)_2F$, $CH_2=CHCOO-CH_2CH_2CH_2(CF)_2H$,
$CH_2=CHCOO-CH_2(CF_2)_4H$, $CH_2=CHCOO-CH_2CH_2(CF_2)_3H$,
$CH_2=CHCOO-CH_2CH_2CF(CF_3)-$, $[OCF_2-CF(CF_3)]_Z-OC_3F_7$,
$CH_2=CHCOO-CH_2CH_2CF_2CF_2(CF_3)-[OCF_2-CF(CF_3)]_2-OC_3F_7$.

Among these, the vinyl polymers represented by the formulae presented below are preferable:
$CH_2=CHCOO-CH_2CH_2(CF_2)_6F$, $CH_2=CHCOO-CH_2CH_2(CF_2)_8F$,
$CH_2=CCH_3COO-CH_2CH_2(CF_2)_6F$, $CH_2=CCH_3COO-CH_2CH_2(CF_2)_8F$,
$CH_2=CHCOO-CH_2CF_3$, $CH_2=CCH_3COO-CH_2CF_3$.

The vinyl polymers represented by the formulae presented below are particularly preferable:
$CH_2=CHCOO-CH_2CF_3$, $CH_2=CCHCOO-CH_2CF_3$.

The vinyl monomers (M2) not containing any organofluorine groups in the molecule may be any monomers containing radical-polymerizable vinyl groups which are exemplified, for example, by methyl acrylate, methyl methacrylate, ethyl acrylate, ethyl methacrylate, n-propyl acrylate, n-propyl methacrylate, isopropyl acrylate, isopropyl methacrylate, and other lower alkyl acrylates or methacrylates; glycidyl acrylate, glycidyl methacrylate; n-butyl acrylate, n-butyl methacrylate, isobutyl acrylate, isobutyl methacrylate, tert-butyl acrylate, tert-butyl methacrylate, n-hexyl acrylate, n-hexyl methacrylate, cyclohexyl acrylate, cyclohexyl methacrylate, 2-ethylhexyl acrylate, 2-ethylhexyl methacrylate, octyl acrylate, octyl methacrylate, lauryl acrylate, lauryl methacrylate, stearyl acrylate, stearyl methacrylate, and other higher acrylates and methacrylates; vinyl acetate, vinyl propionate and other lower fatty acid vinyl esters; vinyl butyrate, vinyl caproate, vinyl 2-ethylhexanoate, vinyl laurate, vinyl stearate, and other higher fatty acid esters; styrene, vinyltoluene, benzyl acrylate, benzyl methacrylate, phenoxyethyl acrylate, phenoxyethyl methacrylate, vinylpyrrolidone, and other vinyl aromatic monomers; dimethylaminoethyl acrylate, dimethylaminoethyl methacrylate, diethylaminoethyl acrylate, diethylaminoethyl methacrylate, and other aminovinyl monomers, acrylamide, methacrylamide, N-methylolacrylamide, N-methylolmethacrylamide, N-methoxymethylacrylamide, N-methoxymethylmethacrylamide, isobutoxymethoxyacrylamide, isobutoxymethoxymethacrylamide, N,N-dimethylacrylamide, N,N-dimethylmethacrylamide, and other vinylamide monomers; hydroxyethyl acrylate, hydroxyethyl methacrylate, acrylic acid hydroxypropyl alcohol, methacrylic acid hydroxypropyl alcohol, and other hydroxyvinyl monomers; acrylic acid, methacrylic acid, itaconic acid, crotonic acid, fumaric acid, maleic acid, and other vinylcarboxylic acid monomers; tetrahydrofurfuryl acrylate, tetrahydrofurfuryl methacrylate, butoxyethyl acrylate, butoxyethyl methacrylate, ethoxydiethylene glycol acrylate, ethoxydiethylene glycol methacrylate, polyethylene glycol acrylate, polyethylene glycol methacrylate, polypropylene glycol monoacrylate, polypropylene glycol mono methacrylate, hydroxybutyl vinyl ether, cetyl vinyl ether, 2-ethylhexyl vinyl ether, and other vinyl monomers containing an ether bond; acryloxypropyltrimethoxysilane, methacryloxypropyltrimethoxysilane, polydimethylsiloxanes containing acryl or methacryl groups at one of the ends, polydimethylsiloxanes containing alkenylaryl groups at one of the ends and other silicone compounds containing unsaturated groups; butadiene; vinyl chloride; vinylidene chloride, acrylonitrile, methacrylonitrile; dibutyl fumarate; maleic anhydride; dodecylsuccinic anhydride; acryl glycidyl ether, methacryl glycidyl ether, 3,4-epoxycyclohexylmethyl acrylate, 3,4-epoxycyclohexylmethyl methacrylate, alkali metal salts, ammonium salts and organic amine salts of acrylic acid, of methacrylic acid, of itaconic acid, of crotonic acid, of fumaric acid, of maleic acid and of other radical-polymerizable unsaturated carboxylic acids, radical-polymerizable unsaturated monomers containing sulfonic acid groups, such as styrene sulfonic acid and also the alkali metal salts thereof, the ammonium salts thereof and the organic amine salts thereof; the quaternary ammonium salts derived from acrylic acid or methacrylic acid, such as 2-hydroxy-3-methacryloxypropyltrimethylammonium chloride, methacrylic acid esters of a tertiary amine alcohol, such as the diethylamine ester of methacrylic acid and quaternary ammonium salts thereof.

In addition, it is also possible to use as vinyl monomers (M2) the polyfunctional vinyl monomers illustrated, for example, by trimethylolpropane triacrylate, trimethylolpropane trimethacrylate, pentaerythrityl triacrylate, pentaerythrityl trimethacrylate, ethylene glycol diacrylate, ethylene glycol dimethacrylate, tetraethylene glycol diacrylate, tetraethylene glycol dimethacrylate, polyethylene glycol diacrylate, polyethylene glycol dimethacrylate, 1,4-butanediol diacrylate, 1,4-butanediol dimethacrylate, 1,6-hexanediol diacrylate, 1,6-hexanediol dimethacrylate, neopentyl glycol diacrylate, neopentyl glycol dimethacrylate, trimethylolpropanetrioxyethyl acrylate, trimethylolpropanetrioxyethyl methacrylate, tris(2-hydroxyethyl)isocyanurate diacrylate, tris(2-hydroxyethyl)isocyanurate dimethacrylate, tris(2-hydroxyethyl)isocyanurate triacrylate, tris(2-hydroxyethyl)isocyanurate trimethacrylate, polydimethylsiloxane in which the two ends of the molecular chain are blocked with alkenylaryl groups, and other silicone compounds containing unsaturated groups.

As regards the ratio mentioned above in which (M1) and (M2) are copolymerized, the weight ratio between (M1) and (M2) is preferably within the range 1:99 to 100:0.

Y can be chosen, for example, from organic groups containing acrylic or methacrylic groups, organic groups containing an alkenylaryl group, or alkenyl groups containing from 2 to 10 carbon atoms.

The organic groups containing acrylic or methacrylic groups and the alkenylaryl groups are as defined above.

Among the compounds (B), mention may, for example, be made of the following compounds:

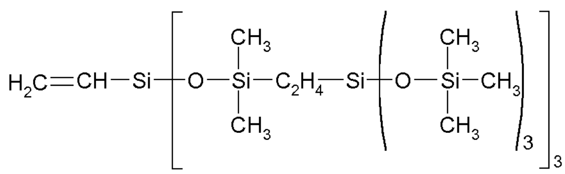

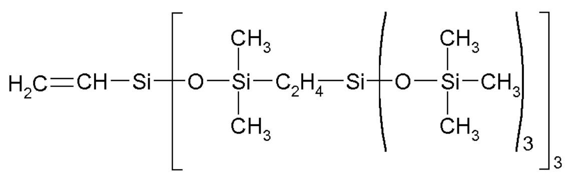

The carbosiloxane dendrimers (B) may be prepared using the process for preparing siloxane/silalkylene branched copolymers described in document EP 1 055 674.

For example, they may be prepared by subjecting organic alkenyl silicone compounds and silicone compounds comprising hydrogen atoms bonded to the silicon, represented by formula (IV) as defined above, to a hydrosilylation reaction.

The copolymerization ratio (by weight) between the monomer (B) and the monomers (M1) and (M2) is preferably within the range of 1:99 to 99:1 and even more preferably within the range of 5:95 to 95:5.

Amino groups may be introduced into the side chains of the vinyl polymer using, included in the component (M2), vinyl monomers containing amino groups, such as dimethylaminoethyl acrylate, dimethylaminoethyl methacrylate, diethylaminoethyl acrylate and diethylaminoethyl methacrylate, followed by performing a modification with potassium acetate monochloride, ammonium acetate monochloride, the aminomethylpropanol salt of monochloroacetic acid, the triethanolamine salt of monobromoacetic acid, sodium monochloropropionate, and other alkali metal salts of halogenated fatty acids; otherwise, carboxylic acid groups may be introduced into the side chains of the vinyl polymer using, included in the component (M2), vinyl monomers containing carboxylic acids, such as acrylic acid, methacrylic acid, itaconic acid, crotonic acid, fumaric acid and maleic acid, and the like, followed by neutralizing the product with triethylamine, diethylamine, triethanolamine and other amines.

A fluorinated vinyl polymer may be one of the polymers described in the examples of patent application WO 03/045 337.

According to one preferred embodiment, a vinyl polymer grafted in the sense of the present invention may be conveyed in an oil or a mixture of oils, which is/are preferably volatile, chosen in particular from silicone oils and hydrocarbon-based oils, and mixtures thereof.

According to one particular embodiment, a silicone oil that is suitable for use in the invention may be cyclopentasiloxane.

According to another particular embodiment, a hydrocarbon-based oil that is suitable for use in the invention may be isododecane.

Vinyl polymers grafted with at least one carbosiloxane dendrimer-based unit that may be particularly suitable for use in the present invention are the polymers sold under the names TIB 4-100, TIB 4-101, TIB 4-120, TIB 4-130, TIB 4-200, FA 4002 ID (TIB 4-202), TIB 4-220 and FA 4001 CM (TIB 4-230) by the company Dow Corning.

According to one embodiment, the composition according to the present invention comprises the vinyl polymer bearing at least one carbosiloxane dendrimer-based unit in an active material content of from 0.5% to 20% and in particular from 1% to 15% by weight, relative to the total weight of the said composition.

## VOLATILE OILS

As indicated previously, the composition according to the invention comprises at least two hydrocarbon-based volatile oils.

Preferably, the hydrocarbon-based volatile oils are apolar.

More particularly, the apolar volatile hydrocarbon-based oil may have a flash point ranging from 40°C to 102°C, preferably ranging from 40°C to 55°C and preferentially ranging from 40°C to 50°C.

The hydrocarbon-based volatile oil may in particular be chosen from hydrocarbon-based volatile oils containing from 8 to 16 carbon atoms, and mixtures thereof, and in particular:

- branched $C_8$-$C_{16}$ alkanes such as $C_8$-$C_{16}$ isoalkanes (also known as isoparaffins), isododecane, isodecane and isohexadecane, and, for example, the oils sold under the trade name Isopar or Permethyl,
- linear $C_8$-$C_{16}$ alkanes, for instance n-dodecane and n-tetradecane sold by Sasol under the respective references Parafol 12-97 and Parafol 14-97, and also mixtures thereof, the undecane-tridecane mixture (Cetiol UT), the mixtures of n-undecane and of n-tridecane obtained in Examples 1 and 2 of patent application WO 2008/155 059 from the company Cognis, and mixtures thereof.

Preferably, the hydrocarbon-based volatile oils are at least chosen from branched alkanes.

In accordance with this embodiment, the content of hydrocarbon-based volatile oil(s) represents from 0.5% to 40% by weight, more particularly from 1% to 30% by weight and preferably from 5% to 30% by weight, relative to the weight of the composition.

The composition according to the invention may also comprise, in addition to the hydrocarbon-based volatile oils, at least one silicone or fluoro volatile oil.

For example, the silicone oils are chosen especially from silicone oils with a flash point ranging from 40°C to 102°C, preferably with a flash point of greater than 55°C and less than or equal to 95°C, and preferentially ranging from 65°C to 95°C.

As volatile silicone oils that may be used, mention may be made of linear or cyclic silicones with a viscosity at room temperature of less than 8 centistokes (cSt) ($8 \times 10^{-6}$ $m^2/s$), and in particular containing from 2 to 10 silicon atoms and in particular from 2 to 7 silicon atoms, these silicones optionally comprising alkyl or alkoxy groups containing from 1 to 10 carbon atoms.
As volatile silicone oils that may be used, mention may be made especially of dimethicones with viscosities of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.
According to another embodiment, the volatile oil is a fluoro oil, such as nonafluoromethoxybutane or perfluoromethylcyclopentane, and mixtures thereof.
Preferably, if one or more other volatile oils are used in addition to the hydrocarbon-based volatile oils, they are chosen from silicone volatile oils.
In addition, if the composition comprises at least one silicone or fluoro volatile oil, preferably a silicone volatile oil, the content thereof is less than 5% by weight relative to the weight of the composition.
More particularly, when the composition comprises at least one silicone volatile oil, the weight ratio of the silicone or fluoro volatile oils, and preferably silicone volatile oils, to the volatile hydrocarbon-based oil(s) is between 0 and 1, limit excluded.
Preferably, the composition according to the invention does not comprise any silicone volatile oil.

## SOLID COMPOUNDS/WAXES

The composition according to the invention may comprise at least one solid compound chosen from solid fatty alcohols, and optionally waxes, in particular polar or apolar hydrocarbon-based waxes, or mixtures thereof.

### Solid fatty alcohols

The composition according to the invention comprises at least one saturated or unsaturated, linear or branched, preferably linear, solid fatty alcohol, whose melting point is greater than or equal to 40°C, comprising from 16 to 60 carbon atoms and advantageously from 18 to 60 carbon atoms. Preferably, the solid fatty alcohol is a monoalcohol.
For example, mention may be made of the wax Performacol 550-L Alcohol from New Phase Technologies, stearyl alcohol, cetyl alcohol, palmityl alcohol, behenyl alcohol, arachidyl alcohol or 1-triacontanol, or mixtures thereof.
Saturated, linear solid fatty alcohols whose melting point is at least 60°C, comprising from 20 to 60 carbon atoms, are preferably used. The content of solid fatty alcohol(s) whose melting point is greater than or equal to 40°C represents from 5% to 20% by weight, preferably from 5% to 15% by weight and more particularly between 7% and 15% by weight, relative to the total weight of the composition.

### Alcohol or alcohol-derived additives

According to an advantageous variant of the present invention, the composition also comprises at least one alcohol or alcohol-derived additive chosen from:

**• solid fatty alcohols with a melting point between 25°C and less than 40°C.**

More particularly, the said alcohols are saturated or unsaturated, preferably saturated, preferably linear, and advantageously comprise at least 14 carbon atoms. Examples that may be mentioned include myristyl alcohol and erucyl alcohol.

**• linear or branched, saturated or unsaturated, monooxyalkylenated or polyoxyalkylenated $C_2$-$C_3$ fatty alcohols containing at least 14 carbon atoms, which are solid at 25°C.**

More particularly, the said fatty alcohols are monooxyethylenated or polyoxyethylenated and advantageously comprise from 14 to 30 and preferably from 16 to 30 carbon atoms. They are preferably linear and saturated.
Preferably, the number of oxyalkylene, preferably oxyethylene, units is between 1 and 100, more particularly between 1 and 50 and preferably between 1 and 30.
Examples that may be mentioned include the ethoxylated derivatives of stearyl alcohol, cetyl alcohol, cetostearyl alcohol, myristyl alcohol or palmityl alcohol, and also mixtures thereof. For example, mention may be made of stearyl alcohol containing 20 mol of ethylene oxide, cetostearyl alcohol containing 20 mol of ethylene oxide or cetostearyl alcohol containing 30 mol of ethylene oxide.

• **liposoluble polyethers resulting from polyetherification between one or more $C_2$-$C_{100}$ and preferably $C_2$-$C_{50}$ diols.**

Among the liposoluble polyethers, the ones particularly under consideration are copolymers of ethylene oxide and/or of propylene oxide with diols comprising a $C_6$-$C_{30}$ alkyl chain.
Preferably, these polyethers have a weight ratio of ethylene oxide and/or propylene oxide relative to the abovementioned diol ranging from 5/95 to 70/30.
Preferably, these compounds are triblock polymers.
In this family, mention will be made especially of copolymers such that the diol(s) comprising an alkyl chain are arranged in blocks with an average molecular weight from 1000 to 10 000, for example polyoxyethylene/polydodecyl glycol block copolymers, such as the ethers of dodecanediol (22 mol) and of polyethylene glycol (45 OE) (INCI name: PEG-45/dodecyl glycol copolymer; sold under the brand name Elfacos ST9 by Akzo Nobel) or the ethers of dodecanediol (9 mol) and of polyethylene glycol (22 OE) (INCI name: PEG-22/dodecyl glycol copolymer; sold under the brand name Elfacos ST37 by Akzo Nobel.

• **or mixtures thereof.**

When the composition comprises one or more of these additives, and this corresponds to a particularly advantageous variant of the invention, the content is between 1% and 10% by weight and preferably between 3% and 7.5% by weight relative to the total weight of the composition.
Preferably, the weight ratio of the abovementioned alcohol or alcohol-derived additive(s)/solid fatty alcohol(s) is less than 1 and preferably between 0 and 1 (limit excluded).

**Waxes**

The composition according to the invention may optionally comprise at least one wax, other than the solid fatty alcohols whose melting point is greater than or equal to 40°C mentioned previously, and other than the alcohol or alcohol-derived additives. More particularly, the said waxes are chosen from polar and apolar hydrocarbon-based waxes, or mixtures thereof.
The wax(es) under consideration in the context of the present invention are generally lipophilic compounds that are solid at room temperature (25°C), with a solid/liquid reversible change of state, having a melting point of greater than or equal to 30°C, which may be up to 200°C and especially up to 120°C.
In particular, the waxes that are suitable for the invention may have a melting point of greater than or equal to 45°C and in particular greater than or equal to 55°C.
For the purposes of the invention, the melting point corresponds to the temperature of the most endothermic peak observed in thermal analysis (DSC) as described in the standard ISO 11357-3; 1999. The melting point of the wax may be measured using a differential scanning calorimeter (DSC), for example the calorimeter sold under the name DSC Q2000 by the company TA Instruments.
Preferably, the waxes have an heat of fusion $\Delta Hf$ of greater than or equal to 70 J/g.
Preferably, the waxes comprise at least one crystallizable part, which is visible by X-ray observation.

<u>The measurement protocol is as follows:</u>

A sample of 5 mg of wax placed in a crucible is subjected to a first temperature rise ranging from -20°C to 120°C, at a heating rate of 10°C/minute, it is then cooled from 120°C to -20°C at a cooling rate of 10°C/minute and is finally subjected to a second temperature increase ranging from -20°C to 120°C at a heating rate of 5°C/minute. During the second temperature increase, the following parameters are measured:

- the melting point (Mp) of the wax, as mentioned previously corresponding to the temperature of the most endothermic peak of the melting curve observed, representing the variation of the difference in power absorbed as a function of the temperature,
- $\Delta Hf$: the heat of fusion of the wax, corresponding to the integral entire melting curve obtained. This heat of fusion of the wax is the amount of energy required to make the compound change from the solid state to the liquid state. It is expressed in J/g.

The wax may especially have a hardness ranging from 0.05 MPa to 15 MPa and preferably ranging from 6 MPa to 15 MPa. The hardness is determined by measuring the compression force, measured at 20°C using the texturometer sold under the name TA-TX2i by the company Rheo, equipped with a stainless-steel cylinder 2 mm in diameter

travelling at a measuring speed of 0.1 mm/s, and penetrating the wax to a penetration depth of 0.3 mm.

If the composition comprises at least one wax, other than the solid fatty alcohols with a melting point of greater than or equal to 40°C mentioned previously, and other than the alcohol or alcohol-derived additives also mentioned hereinabove, their content is preferably such that the wax(es)/solid fatty alcohol(s) weight ratio is less than 1 and more particularly between 0 and 1 (limit excluded).

## Apolar waxes

For the purposes of the present invention, the term "*apolar wax*" means a wax whose solubility parameter at 25°C as defined below, $\delta_a$, is equal to 0 $(J/cm^3)^{½}$.

Apolar waxes are in particular hydrocarbon waxes composed solely of carbon and hydrogen atoms and devoid of heteroatoms, such as N, O, Si and P.

The term "*hydrocarbon-based wax"* means a wax formed essentially from, or even constituted of, carbon and hydrogen atoms, and optionally oxygen and nitrogen atoms, and that does not contain any silicon or fluorine atoms. It may contain alcohol, ester, ether, carboxylic acid, amine and/or amide groups.

More particularly, the apolar wax may be chosen from microcrystalline waxes, paraffin waxes, ozokerite, polyethylene waxes, polymethylene waxes and microwaxes, and mixtures thereof.

As microcrystalline waxes that may be used, mention may be made of Multiwax W 445® sold by the company Sonneborn, and Microwax HW® and Base Wax 30540® sold by the company Paramelt.

An ozokerite that may be mentioned is Ozokerite Wax SP 1020 P.

Polyethylene waxes that may be mentioned include Performalene 500-L Polyethylene and Performalene 400 Polyethylene sold by New Phase Technologies.

Polymethylene waxes that may be mentioned include the Polymethylene Wax (54°C) sold under the reference Cirebelle 303; the Polymethylene Wax (80°C) sold under the reference Cirebelle 108, sold by Cirebelle.

As microwaxes that may be used in the compositions according to the invention as apolar wax, mention may be made especially of polyethylene microwaxes such as those sold under the names Micropoly 200®, 220®, 220L® and 250S® by the company Micro Powders.

Advantageously, if the composition comprises at least one apolar wax, its content represents from 0.1% to 5% by weight relative to the weight of the composition.

## Polar waxes

For the purposes of the present invention, the term "polar wax" means a wax whose solubility parameter at 25°C, $\delta_a$, is other than 0 $(J/cm^3)^{½}$.

In particular, the term "*polar wax*" means a wax whose chemical structure is formed essentially from, or even constituted of, carbon and hydrogen atoms, and comprising at least one highly electronegative heteroatom such as an oxygen, nitrogen, silicon or phosphorus atom.

The definition and calculation of the solubility parameters in the Hansen three-dimensional solubility space are described in the article by C.M. Hansen: The three-dimensional solubility parameters, J. Paint Technol. 39, 105 (1967).

According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{½}$

The parameters $\delta_p$, $\delta_h$, $\delta_D$ and $\delta_a$ are expressed in $(J/cm^3)^{½}$.

According to one variant of the invention, the polar wax is a hydrocarbon-based wax.

As hydrocarbon-based polar wax, a wax chosen from ester waxes is in particular preferred.

The term "*hydrocarbon-based*" means a compound formed essentially from, or even constituted of, carbon and hydrogen atoms, and optionally oxygen and nitrogen atoms, and that does not contain any silicon or fluorine atoms.

According to the invention, the term "*ester wax*" means a wax comprising at least one ester function.

The following may especially be used as ester wax:

- ester waxes such as those chosen from:

i) waxes of formula $R_1COOR_2$ in which $R_1$ and $R_2$ represent linear, branched or cyclic aliphatic chains in which the number of atoms ranges from 10 to 50, which may contain a heteroatom such as O, N or P and whose melting point ranges from 25 to 120°C.
In particular, use may be made, as ester wax, of a $C_{20}$-$C_{40}$ alkyl (hydroxystearyloxy)stearate (the alkyl group containing from 20 to 40 carbon atoms), alone or as a mixture, or a $C_{20}$-$C_{40}$ alkyl stearate. Such waxes are especially sold under the names Kester Wax K 82 P®, Hydroxypolyester K 82 P®, Kester Wax K 80 P® and Kester Wax K82H by the company Koster Keunen.

ii) glycol and butylene glycol montanate (octacosanoate) waxes such as the wax Licowax KPS Flakes (INCI name: glycol montanate) sold by the company Clariant.

iii) bis(1,1,1-trimethylolpropane) tetrastearate, sold under the name Hest 2T-4S® by the company Heterene.

iv) diester waxes of a dicarboxylic acid of general formula $R^3$-(-OCO-$R^4$-COO-$R^5$), in which $R^3$ and $R^5$ are identical or different, preferably identical and represent a $C_4$-$C_{30}$ alkyl group (alkyl group comprising from 4 to 30 carbon atoms) and $R^4$ represents a linear or branched $C_4$-$C_{30}$ aliphatic group (alkyl group comprising from 4 to 30 carbon atoms) which may or may not contain one or more unsaturated groups, and preferably that is linear and unsaturated.

v) Mention may also be made of the waxes obtained by catalytic hydrogenation of animal or vegetable oils having linear or branched $C_8$-$C_{32}$ fatty chains, for example such as hydrogenated jojoba oil, hydrogenated sunflower oil, hydrogenated castor oil, hydrogenated coconut oil, and also the waxes obtained by hydrogenation of castor oil esterified with cetyl alcohol, such as those sold under the names Phytowax Ricin 16L64® and 22L73® by the company Sophim. Such waxes are described in patent application FR-A-2792190 and the waxes obtained by hydrogenation of olive oil esterified with stearyl alcohol such as that sold under the name Phytowax Olive 18 L 57, or else.

vi) beeswax, synthetic beeswax, polyglycerolated beeswax, carnauba wax, candelilla wax, oxypropylenated lanolin wax, rice bran wax, ouricury wax, esparto grass wax, cork fibre wax, sugar cane wax, Japan wax, sumach wax, montan wax, orange wax, laurel wax and hydrogenated jojoba wax. Candelilla wax is preferably used.

According to a particular embodiment of the invention, the composition comprises at least one polar wax. Advantageously, the polar wax(es) are chosen from hydrocarbon-based polar waxes, more particularly from ester waxes, and preferably the abovementioned waxes vi), and mixtures thereof.

In accordance with a particular embodiment of the invention, when it is present, the content of polar wax(es) in the composition is between 0.1% and 5% by weight and preferably between 0.1% and 2% by weight, relative to the total weight of the composition.

Preferably, the composition comprises at least one solid fatty alcohol as described previously.

Furthermore, it very advantageously comprises at least one abovementioned alcohol or alcohol-derived additive.

In accordance with another variant of the invention, the composition comprises, besides the solid fatty alcohols and the alcohol or alcohol-derived additive, at least one polar wax, preferably chosen from the waxes vi).

## PASTY FATTY SUBSTANCES

The composition according to the invention may also comprise at least one pasty fatty substance.

For the purposes of the present invention, the term "pasty fatty substance" means a lipophilic fatty compound that undergoes a reversible solid/liquid change in state, which exhibits, in the solid state, an anisotropic crystalline arrangement and which comprises, at a temperature of 23°C, a liquid fraction and a solid fraction.

In other words, the starting melting point of the pasty fatty substance can be less than 23°C. The liquid fraction of the pasty fatty substance measured at 23°C can represent from 9% to 97% by weight of the pasty fatty substance. This liquid fraction at 23°C preferably represents between 15% and 85% and more preferably between 40% and 85% by weight.

For the purposes of the invention, the melting point corresponds to the temperature of the most endothermic peak observed in thermal analysis (DSC) as described in the standard ISO 11357-3; 1999. The melting point of a pasty fatty substance may be measured using a differential scanning calorimeter (DSC), for example the calorimeter sold under the name MDSC 2920 by the company TA Instruments.

The measurement protocol is as follows:

A sample of 5 mg of pasty fatty substance placed in a crucible is subjected to a first temperature rise ranging from -20°C to 100°C, at a heating rate of 10°C/minute, is then cooled from 100°C to -20°C at a cooling rate of 10°C/minute and is finally subjected to a second temperature rise ranging from -20°C to 100°C at a heating rate of 5°C/minute. During the second temperature rise, the variation in the difference in power absorbed by the empty crucible and by the crucible containing the sample of pasty fatty substance is measured as a function of the temperature. The melting point of the pasty fatty substance is the value of the temperature corresponding to the tip of the peak of the curve representing the variation in the difference in power absorbed as a function of the temperature.

The liquid fraction by weight of the pasty fatty substance at 23°C is equal to the ratio of the heat of fusion consumed at 23°C to the heat of fusion of the pasty fatty substance.

The heat of fusion of the pasty fatty substance is the heat consumed by the compound in order to pass from the solid state to the liquid state. The pasty fatty substance is said to be in the solid state when all of its mass is in crystalline solid form. The pasty fatty substance is said to be in the liquid state when all of its mass is in liquid form.

The heat of fusion of the pasty fatty substance is equal to the area under the curve of the thermogram obtained using a differential scanning calorimeter (DSC), such as the calorimeter sold under the name MDSC 2920 by the company TA Instrument, with a temperature rise of 5°C or 10°C per minute, according to Standard ISO 11357-3; 1999.

The heat of fusion of the pasty fatty substance is the amount of energy required to make the pasty fatty substance change from the solid state to the liquid state. It is expressed in J/g.

The heat of fusion consumed at 23°C is the amount of energy absorbed by the sample to change from the solid state to the state that it has at 23°C, composed of a liquid fraction and a solid fraction.

The liquid fraction of the pasty fatty substance measured at 32°C preferably represents from 30% to 100% by weight of the pasty fatty substance, preferably from 50% to 100%, more preferably from 60% to 100% by weight of the pasty fatty substance. When the liquid fraction of the pasty fatty substance measured at 32°C is equal to 100%, the temperature of the end of the melting range of the pasty fatty substance is less than or equal to 32°C.

The liquid fraction of the pasty fatty substance measured at 32°C is equal to the ratio of the heat of fusion consumed at 32°C to the heat of fusion of the pasty fatty substance. The heat of fusion consumed at 32°C is calculated in the same way as the heat of fusion consumed at 23°C.

The pasty fatty substance may in particular be chosen from synthetic fatty substances and fatty substances of vegetable origin. A pasty fatty substance may be obtained by synthesis from starting materials of vegetable origin.

The pasty fatty substance may be chosen from:

- lanolin and derivatives thereof,
- petroleum jelly (also known as petrolatum),
- polyol ethers chosen from pentaerythrityl ethers of a polyalkylene glycol, fatty alkyl ethers of a sugar, and mixtures thereof, the pentaerythrityl ether of polyethylene glycol comprising 5 oxyethylene units (5 OE) (CTFA name: PEG-5 Pentaerythrityl Ether), polypropylene glycol pentaerythrityl ether comprising five oxypropylene (5 OP) units (CTFA name: PPG-5 Pentaerythrityl Ether) and mixtures thereof, and more especially the mixture PEG-5 Pentaerythrityl Ether, PPG-5 Pentaerythrityl Ether and soybean oil, sold under the name Lanolide by the company Vevy, which is a mixture in which the constituents are in a 46/46/8 weight ratio: 46% PEG-5 Pentaerythrityl Ether, 46% PPG-5 Pentaerythrityl Ether and 8% soybean oil,
- polymeric or non-polymeric silicone compounds,
- polymeric or non-polymeric fluoro compounds,
- vinyl polymers, especially:
    - olefin homopolymers and copolymers,
    - hydrogenated diene homopolymers and copolymers,
    - linear or branched oligomers, homopolymers or copolymers of alkyl (meth)acrylates preferably containing a $C_8$-$C_{30}$ alkyl group,
    - oligomers, which are homopolymers and copolymers of vinyl esters containing $C_8$-$C_{30}$ alkyl groups, and
    - oligomers, which are homopolymers and copolymers of vinyl ethers containing $C_8$-$C_{30}$ alkyl groups,
- esters,
- and/or mixtures thereof.

Among the esters, the following are especially considered:

- esters of a glycerol oligomer, especially diglycerol esters, in particular condensates of adipic acid and of glycerol,

for which some of the hydroxyl groups of the glycerols have reacted with a mixture of fatty acids such as stearic acid, capric acid, isostearic acid and 12-hydroxystearic acid, such as, for example, bis-diglyceryl polyacyladipate-2 sold under the reference Softisan® 649 by the company Sasol,

- vinyl ester homopolymers containing $C_8$-$C_{30}$ alkyl groups, such as polyvinyl laurate (sold especially under the reference Mexomer PP by the company Chimex),
- the arachidyl propionate sold under the brand name Waxenol 801 by Alzo,
- phytosterol esters,
- fatty acid triglycerides and derivatives thereof,
- pentaerythritol esters,
- esters of a diol dimer and of a diacid dimer, where appropriate esterified on their free alcohol or acid function(s) with acid or alcohol radicals, especially dimer dilinoleate esters; such esters may be chosen especially from the esters having the following INCI nomenclature: bis-behenyl/isostearyl/phytosteryl dimer dilinoleyl dimer dilinoleate (Plandool G), phytosteryl/isostearyl/cetyl/stearyl/behenyl dimer dilinoleate (Plandool H or Plandool S), and mixtures thereof,
- butters of plant origin, such as mango butter, such as the product sold under the reference Lipex 203 by the company Aarhuskarlshamn, shea butter, in particular the product whose INCI name is Butyrospermum Parkii Butter, such as the product sold under the reference Sheasoft® by the company Aarhuskarlshamn, cupuacu butter (Rain Forest RF3410 from the company Beraca Sabara), murumuru butter (Rain Forest RF3710 from the company Beraca Sabara), cocoa butter; and also orange wax, for instance the product sold under the reference Orange Peel Wax by the company Koster Keunen,
- totally or partially hydrogenated plant oils, for instance hydrogenated soybean oil, hydrogenated coconut oil, hydrogenated rapeseed oil, mixtures of hydrogenated plant oils such as the mixture of hydrogenated soybean, coconut, palm and rapeseed plant oil, for example the mixture sold under the reference Akogel® by the company Aarhuskarlshamn (INCI name Hydrogenated Vegetable Oil), the trans-isomerized partially hydrogenated jojoba oil manufactured or sold by the company Desert Whale under the commercial reference Iso-Jojoba-50®, partially hydrogenated olive oil, for instance the compound sold under the reference Beurrolive by the company Soliance,
- hydrogenated castor oil esters, such as hydrogenated castor oil dimer dilinoleate, for example Risocast DA-L sold by Kokyu Alcohol Kogyo, and hydrogenated castor oil isostearate, for example Salacos HCIS (V-L) sold by Nisshin Oil,
- and mixtures thereof.

According to a preferred embodiment, the pasty fatty substance is chosen from esters and in particular diglycerol esters, and mixtures thereof.

Among the pasty compounds, bis-behenyl/isostearyl/phytosteryl dimer dilinoleyl, bis(diglyceryl) poly(2-acyladipate), hydrogenated castor oil dimer dilinoleate, for example Risocast DA-L sold by Kokyu Alcohol Kogyo, and hydrogenated castor oil isostearate, for example Salacos HCIS (V-L) sold by Nisshin Oil, polyvinyl laurate, mango butter, shea butter, hydrogenated soybean oil, hydrogenated coconut oil and hydrogenated rapeseed oil, or a mixture thereof, will preferably be chosen.

Advantageously, if the composition comprises at least one pasty fatty substance, then the content thereof represents from 0.1% to 20% by weight relative to the total weight of the composition.

## NON-VOLATILE SILICONE OILS

According to a variant of the invention, the composition preferably comprises at least one phenyl silicone non-volatile oil, with at least one dimethicone fragment, at least one phenyl silicone non-volatile oil not containing any dimethicone fragments, or mixtures thereof.

### Phenyl silicone non-volatile oils not containing any dimethicone fragments

The composition according to the invention comprises at least one non-volatile phenyl silicone first oil not containing any dimethicone fragments.

The term "silicone oil" means an oil containing at least one silicon atom, and in particular containing Si-O groups.

The term "phenyl" specifies that the said oil comprises in its structure at least one phenyl radical.

The term "dimethicone fragment" denotes a divalent siloxane group in which the silicon atom bears two methyl radicals, this group not being located at the ends of the molecule. It may be represented by the following formula: $-(Si(CH_3)_2-O)-$.

The term "non-volatile" is intended to mean an oil of which the vapour pressure at 25°C and atmospheric pressure is non-zero and is less than 0.02 mmHg (2.66 Pa) and better still less than $10^{-3}$ mmHg (0.13 Pa).

As non-volatile phenyl silicone first oil not containing any dimethicone fragments, which is suitable for use in

the present invention, mention may be made of the following oils, alone or as mixtures:

**a)** the phenyl silicone oils corresponding to formula (I) below:

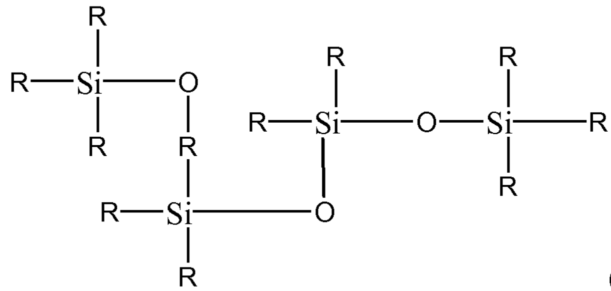

(I)

in which the groups R, which are monovalent or divalent, represent, independently of each other, a methyl, methylene, phenyl or phenylene, with the proviso that at least one group R represents a phenyl and that formula (I) does not comprise any dimethicone fragments.

Preferably, in this formula, the phenyl silicone oil comprises at least three, for example at least four, at least five or at least six, phenyl groups.

**b)** the phenyl silicone oils corresponding to formula (II) below:

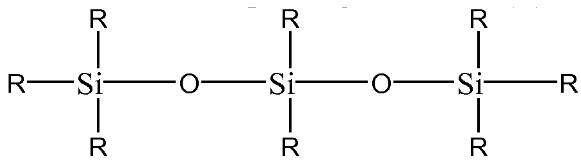

(II)

in which the groups R represent, independently of each other, a methyl or a phenyl, with the proviso that at least one group R represents a phenyl and that formula (I) does not comprise any dimethicone fragments.

Preferably, in this formula, the compound of formula (II) comprises at least three, for example at least four or at least five, phenyl groups.

Mixtures of different phenylorganopolysiloxane compounds described above can be used.

Examples that may be mentioned include mixtures of triphenyl-, tetraphenyl- or pentaphenyl-organopolysiloxanes.

Among the compounds of formula (II), mention may be made more particularly of phenyl silicone oils not containing a dimethicone fragment, corresponding to formula (II) in which at least 4 or at least 5 radicals R represent a phenyl radical, the remaining radicals representing methyls.

Such non-volatile phenyl silicone oils are preferably trimethylpentaphenyltrisiloxane or tetramethyltetraphenyltrisiloxane. They are in particular sold by Dow Corning under the reference PH-1555 HRI or Dow Corning 555 Cosmetic Fluid (chemical name: 1,3,5-trimethyl-1,1,3,5,5-pentaphenyltrisiloxane; INCI name: trimethylpentaphenyltrisiloxane), or the tetramethyltetraphenyltrisiloxane sold under the reference Dow Corning 554 Cosmetic Fluid by Dow Corning can also be used.

They correspond especially to formulae (IIa) and (IIb) below:

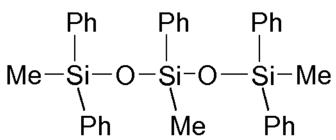

(IIa)

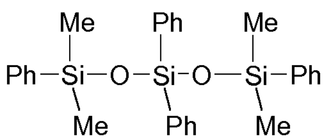

(IIb)

in which Me represents methyl, and Ph represents phenyl.

**c)** the phenyl silicone oils corresponding to formula (III) below:

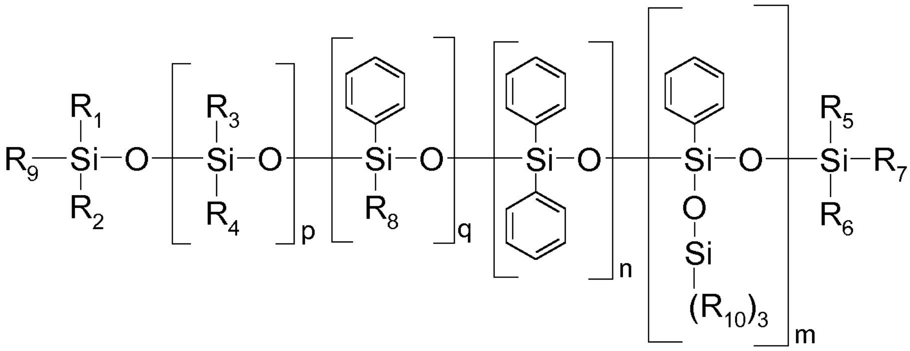

(III)

in which:

- $R_1$ to $R_{10}$, independently of each other, are saturated or unsaturated, linear, cyclic or branched $C_1$-$C_{30}$ hydrocarbon-based radicals,
- m, n, p and q are, independently of each other, integers between 0 and 900, with the proviso that the sum m+n+q is other than 0 and that p is equal to 0 if $R_3$ and $R_4$ represent methyl groups.

Preferably, the sum m+n+q is between 1 and 100. Advantageously, the sum m+n+p+q is between 1 and 900 and preferably between 1 and 800.

Preferably, q is equal to 0.

More particularly, $R_1$ to $R_{10}$, independently of each other, represent a saturated or unsaturated, preferably saturated, linear or branched $C_1$-$C_{30}$ hydrocarbon-based radical, and in particular a preferably saturated, $C_1$-$C_{20}$, in particular $C_1$-$C_{18}$, hydrocarbon-based radical, or a monocyclic or polycyclic $C_6$-$C_{14}$, and in particular $C_{10}$-$C_{13}$, aryl radical, or an aralkyl radical, the alkyl part of which is preferably $C_1$-$C_3$ alkyl.

Preferably, $R_1$ to $R_{10}$ may each represent a methyl, ethyl, propyl, butyl, isopropyl, decyl, dodecyl or octadecyl radical, or alternatively a phenyl, tolyl, benzyl or phenethyl radical. $R_1$ to $R_{10}$ may in particular be identical, and in addition may be a methyl radical.

According to a first more particular embodiment of formula (III), mention may be made of:

**i)** the phenyl silicone oils corresponding to formula (IIIi) below:

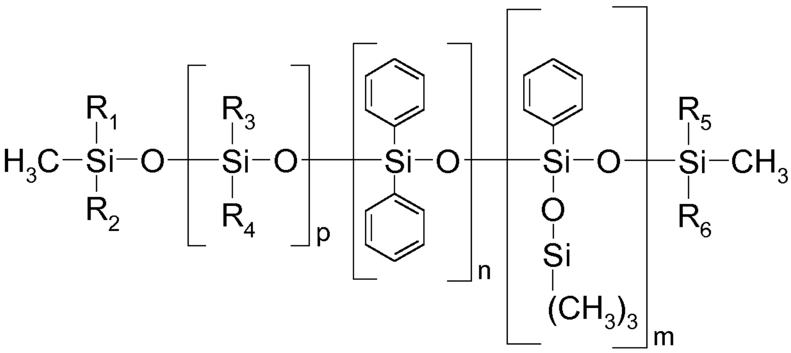

(IIIi)

in which:

- $R_1$ to $R_6$, independently of each other, are saturated or unsaturated, linear, cyclic or branched $C_1$-$C_{30}$ hydrocarbon-

based radicals, a preferably $C_6$-$C_{14}$ aryl radical or an aralkyl radical, the alkyl part of which is $C_1$-$C_3$ alkyl,

- m, n and p are, independently of each other, integers between 0 and 100, with the proviso that the sum n+m is between 1 and 100 and that p is equal to 0 if $R_3$ and $R_4$ represent methyl groups.

Preferably, $R_1$ to $R_6$, independently of each other, represent a $C_1$-$C_{20}$, in particular $C_1$-$C_{18}$, hydrocarbon-based, preferably alkyl, radical, or a $C_6$-$C_{14}$ aryl radical which is monocyclic (preferably $C_6$) or polycyclic and in particular $C_{10}$-$C_{13}$, or an aralkyl radical (preferably the aryl part is $C_6$ aryl; the alkyl part is $C_1$-$C_3$ alkyl).

Preferably, $R_1$ to $R_6$ may each represent a methyl, ethyl, propyl, butyl, isopropyl, decyl, dodecyl or octadecyl radical, or alternatively a phenyl, tolyl, benzyl or phenethyl radical.

$R_1$ to $R_6$ may in particular be identical, and in addition may be a methyl radical. Preferably, m = 1 or 2 or 3, and/or n = 0 and p = 0 can be applied, in formula (IIIi).

According to a suitable variant, mention may be made of compounds (B) derived from formula (IIIi) below:

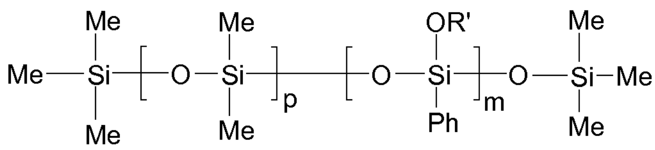

(B)

in which Me is methyl and Ph is phenyl, OR' represents a group $-OSiMe_3$, p is equal to 0 and m is between 1 and 1000. In particular, m and p are such that compound (B) is a non-volatile oil.

Phenyltrimethylsiloxytrisiloxane, sold in particular under the reference Dow Corning 556 Cosmetic Grade Fluid (DC556), may, for example, be used.

**ii)** non-volatile phenyl silicone oils not containing any dimethicone fragments corresponding to formula (IIIii) below:

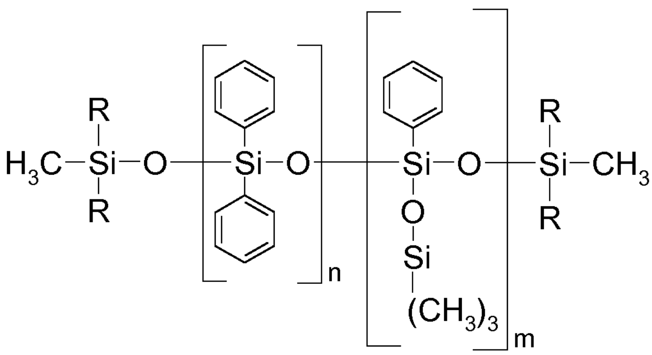

(IIIii)

in which:

- R, independently of each other, are saturated or unsaturated, linear, cyclic or branched $C_1$-$C_{30}$ hydrocarbon-based radicals, preferably R is a $C_1$-$C_{30}$ alkyl radical, a preferably $C_6$-$C_{14}$ aryl radical, or an aralkyl radical, the alkyl part of which is $C_1$-$C_3$ alkyl,
- m and n are, independently of each other, integers between 0 and 100, with the proviso that the sum n+m is between 1 and 100.

Preferably, R, independently of each other, represent a saturated or unsaturated, preferably saturated, linear or branched $C_1$-$C_{30}$ hydrocarbon-based radical, and in particular a preferably saturated, $C_1$-$C_{20}$, in particular $C_1$-$C_{18}$ and more particularly $C_4$-$C_{10}$, hydrocarbon-based radical, a monocyclic or polycyclic $C_6$-$C_{14}$, and in particular $C_{10}$-$C_{13}$, aryl radical, or an aralkyl radical of which preferably the aryl part is $C_6$ aryl and the alkyl part is $C_1$-$C_3$ alkyl.

Preferably, the Rs may each represent a methyl, ethyl, propyl, butyl, isopropyl, decyl, dodecyl or octadecyl radical, or alternatively a phenyl, tolyl, benzyl or phenethyl radical.

The Rs may in particular be identical, and in addition may be a methyl radical.

Preferably, m = 1 or 2 or 3, and/or n = 0 and/or p = 0 or 1 can be applied, in formula (IIIii).

According to a preferred embodiment, n is an integer between 0 and 100 and m is an integer between 1 and 100, with the proviso that the sum n+m is between 1 and 100, in formula (IIIii). Preferably, R is a methyl radical.

According to one embodiment, a phenyl silicone oil of formula (IIIii) with a viscosity at 25°C of between 5 and 1500 $mm^2/s$ (i.e. 5 to 1500 cSt), and preferably with a viscosity of between 5 and 1000 $mm^2/s$ (i.e. 5 to 1000 cSt), may be used.

According to this embodiment, the non-volatile phenyl silicone oil is preferably chosen from phenyl trimethicones (when n=0) such as DC556 from Dow Corning (22.5 cSt), or else from diphenylsiloxyphenyl trimethicone oil (when m and n are between 1 and 100) such as KF56 A from Shin Etsu, or the Silbione 70663V30 oil from Rhône-Poulenc (28 cSt). The values in parentheses represent the viscosities at 25°C.

d) the phenyl silicone oils corresponding to formula (IV) below:

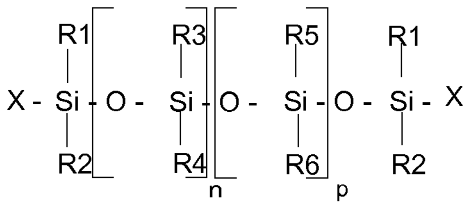

(IV)

in which:

$R_1$, $R_2$, $R_5$ and $R_6$, which may be identical or different, are an alkyl radical containing 1 to 6 carbon atoms, $R_5$ and $R_6$ not simultaneously representing a methyl radical,
$R_3$ and $R_4$, which may be identical or different, are an alkyl radical containing from 1 to 6 carbon atoms or an aryl radical (preferably $C_6$-$C_{14}$), with the proviso that at least one of $R_3$ and $R_4$ is a phenyl radical,
X is an alkyl radical containing from 1 to 6 carbon atoms, a hydroxyl radical or a vinyl radical,
n and p being an integer greater than or equal to 1, chosen so as to give the oil a weight-average molecular weight of less than 200 000 g/mol, preferably less than 150 000 g/mol and more preferably less than 100 000 g/mol.

Preferably, the first oil is chosen from the oils of formula (II) or (III), and mixtures thereof, and even more preferably from the phenyl silicone oils of formulae (IIa) and (IIIi), in particular formulae (B) and (IIIii), and also mixtures thereof.

When the composition comprises such phenyl silicone non-volatile oil(s) without dimethicone fragments, the content thereof is between 10% and 50% by weight and preferably from 15% to 40% by weight relative to the weight of the composition.

**Phenyl silicone non-volatile oils comprising at least one dimethicone fragment**

According to another variant of the invention, the composition comprises at least one phenyl silicone non-volatile oil containing at least one dimethicone fragment.

The definitions recalled in the context of the description of the phenyl silicone oils without dimethicone fragments remain valid and will not be repeated.

As phenyl silicone non-volatile oil containing at least one dimethicone fragment, which is suitable for use in the present invention, mention may be made of the following oils, alone or as mixtures:

**a)** the phenyl silicone oils corresponding to formula (I') below:

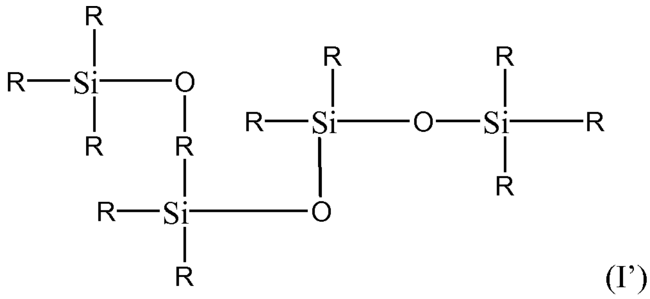

(I')

in which the groups R, which are monovalent or divalent, represent, independently of each other, a methyl, methylene, phenyl or phenylene, with the proviso that at least one group R represents a phenyl and that formula (I') comprises at least one dimethicone fragment.

Preferably, in this formula, the phenyl silicone oil comprises at least three, for example at least four, at least five or at least six, phenyl groups.

**b)** the phenyl silicone oils corresponding to formula (II') below:

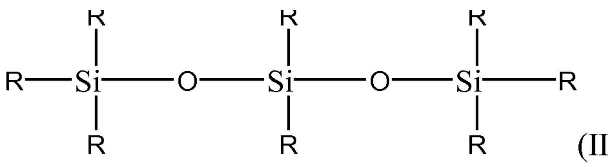

(II')

in which the groups R represent, independently of each other, a methyl or a phenyl, with the proviso that at least one group R represents a phenyl and that formula (II') comprises at least one dimethicone fragment.

Preferably, in this formula, the compound of formula (II') comprises at least three, for example at least four or at least five, phenyl groups.

Mixtures of different phenylorganopolysiloxane compounds described above can be used.

Examples that may be mentioned include mixtures of triphenyl-, tetraphenyl- or pentaphenyl-organopolysiloxanes.

**c)** the phenyl silicone oils corresponding to formula (III') below:

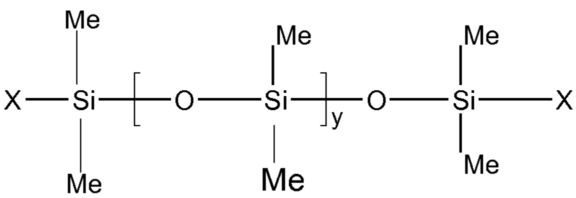

(III')

in which Me represents methyl, y is between 1 and 1000 and X represents $-CH_2-CH(CH_3)(Ph)$.

**d)** the phenyl silicone oils corresponding to formula (IV') below:

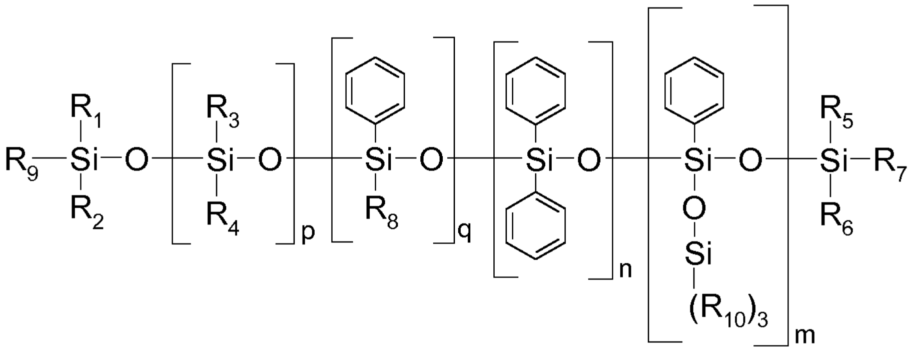

(IV')

in which:

- $R_1$ to $R_{10}$, independently of each other, are saturated or unsaturated, linear, cyclic or branched $C_1$-$C_{30}$ hydrocarbon-based radicals,
- m, n, p and q are, independently of each other, integers between 0 and 900, with the proviso that the sum m+n+q is other than 0;
- formula (IV') comprising at least one dimethicone fragment.

Preferably, the sum m+n+q is between 1 and 100. Advantageously, the sum m+n+p+q is between 1 and 900 and preferably between 1 and 800.

Preferably, q is equal to 0.

More particularly, $R_1$ to $R_{10}$, independently of each other, represent a saturated or unsaturated, preferably saturated, linear or branched $C_1$-$C_{30}$ hydrocarbon-based radical, and in particular a preferably saturated, $C_1$-$C_{20}$, in particular $C_1$-$C_{18}$, hydrocarbon-based radical, or a monocyclic or polycyclic $C_6$-$C_{14}$, and in particular $C_{10}$-$C_{13}$, aryl radical, or an aralkyl radical, the alkyl part of which is preferably $C_1$-$C_3$ alkyl.

Preferably, $R_1$ to $R_{10}$ may each represent a methyl, ethyl, propyl, butyl, isopropyl, decyl, dodecyl or octadecyl radical, or alternatively a phenyl, tolyl, benzyl or phenethyl radical. $R_1$ to $R_{10}$ may in particular be identical, and in addition may be a methyl radical.

According to a more particular embodiment of formula (IV'), mention may be made of the phenyl silicone oils corresponding to formula (IV'i) below:

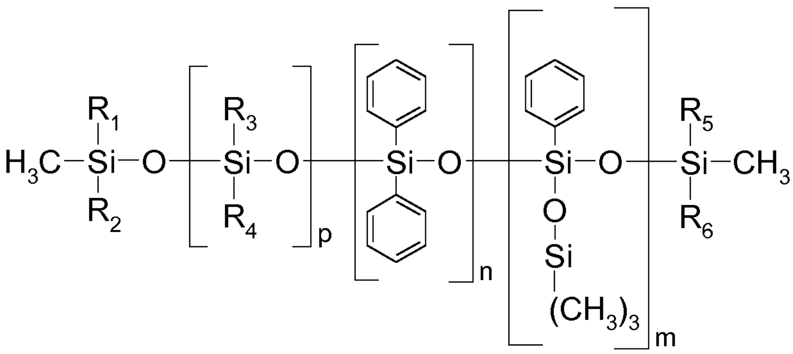

(IV'i)

in which:

- $R_1$ to $R_6$, independently of each other, are saturated or unsaturated, linear, cyclic or branched $C_1$-$C_{30}$ hydrocarbon-based radicals, a preferably $C_6$-$C_{14}$ aryl radical or an aralkyl radical, the alkyl part of which is $C_1$-$C_3$ alkyl,
- m, n and p are, independently of each other, integers between 0 and 100, with the proviso that the sum n+m is between 1 and 100,
- formula (IV'i) comprising at least one dimethicone fragment.

Preferably, $R_1$ to $R_6$, independently of each other, represent a $C_1$-$C_{20}$, in particular $C_1$-$C_{18}$, hydrocarbon-based,

preferably alkyl, radical, or a $C_6$-$C_{14}$ aryl radical which is monocyclic (preferably $C_6$) or polycyclic and in particular $C_{10}$-$C_{13}$, or an aralkyl radical (preferably the aryl part is $C_6$ aryl; the alkyl part is $C_1$-$C_3$ alkyl); formula (IV'i) comprising at least one dimethicone fragment.

Preferably, $R_1$ to $R_6$ may each represent a methyl, ethyl, propyl, butyl, isopropyl, decyl, dodecyl or octadecyl radical, or alternatively a phenyl, tolyl, benzyl or phenethyl radical; formula (IV'i) comprising at least one dimethicone fragment.

$R_1$ to $R_6$ may in particular be identical, and in addition may be a methyl radical. Preferably, m = 1 or 2 or 3, and/or n = 0 and/or p = 0 or 1 can be applied, in formula (IV'i).

Preferably, the phenyl silicone oils that may be used as second oil in the context of the invention correspond to compounds of formula (IV'i) in which:

**A)** m=0 and n and p are, independently of each other, integers between 1 and 100.

Preferably, $R_1$ to $R_6$ are methyl radicals.

According to this embodiment, the silicone oil is preferably chosen from a diphenyl dimethicone such as KF-54 from Shin Etsu (400 cSt), KF54HV from Shin Etsu (5000 cSt), KF-50-300CS from Shin Etsu (300 cSt), KF-53 from Shin Etsu (175 cSt) or KF-50-100CS from Shin Etsu (100 cSt).

**B)** p is between 1 and 100, the sum n+m is between 1 and 100, and n=0.

These phenyl silicone oils optionally containing at least one dimethicone fragment correspond more particularly to formula (B) below:

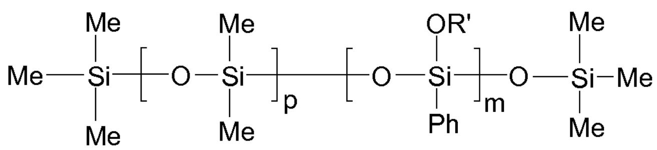

(B)

in which Me is methyl and Ph is phenyl, OR' represents a group $-OSiMe_3$ and p is between 1 and 1000, and m is between 1 and 1000. In particular, m and p are such that compound (B) is a non-volatile oil.

According to a particular embodiment, the non-volatile phenyl silicone oil is such that p is between 1 and 1000 and m is more particularly such that compound (B) is a non-volatile oil. Trimethylsiloxyphenyldimethicone, sold in particular under the reference Belsil PDM 1000 by the company Wacker, may, for example, be used.

**e)** the phenyl silicone oils corresponding to formula (V') below:

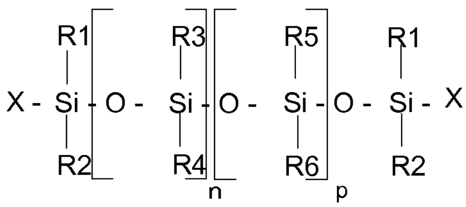

(V')

in which:

$R_1$, $R_2$, $R_5$ and $R_6$, which may be identical or different, are an alkyl radical containing 1 to 6 carbon atoms,
$R_3$ and $R_4$, which may be identical or different, are an alkyl radical containing from 1 to 6 carbon atoms or an aryl radical (preferably $C_6$-$C_{14}$), with the proviso that at least one of $R_3$ and $R_4$ is a phenyl radical,
X is an alkyl radical containing from 1 to 6 carbon atoms, a hydroxyl radical or a vinyl radical,
n and p being an integer greater than or equal to 1, chosen so as to give the oil a weight-average molecular weight of less than 200 000 g/mol, preferably less than 150 000 g/mol and more preferably less than 100 000 g/mol,

- formula (V') comprising at least one dimethicone fragment.

Preferably, the second oil is chosen from the oils of formula (IV'), more particularly of formula (IV'i) and preferably the oils in accordance with variants (A) and (B), and also mixtures thereof.
When the composition comprises at least one non-volatile phenyl silicone oil, the content thereof is between 5% and 20% by weight and preferably from 5% to 15% by weight relative to the weight of the composition.
In accordance with a particularly advantageous embodiment, the composition comprises at least one phenyl silicone oil not containing any dimethicone fragments and at least one phenyl silicone oil containing at least one dimethicone fragment.

## ADDITIONAL NON-VOLATILE OILS

The composition according to the invention may also comprise at least one additional non-volatile oil.
More particularly, this or these additional non-volatile oil(s) may be chosen from polar or apolar hydrocarbon-based non-volatile oils or from non-phenyl silicone non-volatile oils, and also mixtures thereof.

### Polar non-volatile hydrocarbon-based oils

The term "hydrocarbon-based oil" is intended to mean an oil formed essentially from, or even constituted by, carbon and hydrogen atoms, and possibly oxygen and nitrogen atoms, and not containing any silicon or fluorine atoms.
It may contain alcohol, ester, ether, carboxylic acid, amine and/or amide groups.
Preferably, the hydrocarbon-based oil, in addition to being free of silicon and fluorine, is free of heteroatoms such as N and P. The hydrocarbon-based oil is therefore different from a silicone oil and from a fluoro oil.
In the present case, the non-volatile hydrocarbon-based oil comprises at least one oxygen atom.
The term "non-volatile" is intended to mean an oil of which the vapour pressure at 25°C and atmospheric pressure is non-zero and is less than 0.02 mmHg (2.66 Pa) and better still less than $10^{-3}$ mmHg (0.13 Pa).
In particular, this non-volatile hydrocarbon-based oil comprises at least one alcohol function (it is then an "alcohol oil") or at least one ester function (it is then an "ester oil").
The ester oils that may be used in the compositions according to the invention may in particular be hydroxylated.
The composition may comprise one or more non-volatile hydrocarbon-based oils, in particular chosen from:

- <u>$C_{10}$-$C_{26}$ alcohols</u>, preferably monoalcohols.

More particularly, the $C_{10}$-$C_{26}$ alcohols are saturated or unsaturated, and branched or unbranched, and comprise from 10 to 26 carbon atoms.
Preferably, the $C_{10}$-$C_{26}$ alcohols are fatty alcohols, which are preferably branched when they comprise at least 16 carbon atoms.
As examples of fatty alcohols that may be used according to the invention, mention may be made of linear or branched fatty alcohols, of synthetic origin or alternatively of natural origin, for example alcohols derived from plant material (coconut, palm kernel, palm, etc.) or animal material (tallow, etc.).
Needless to say, other long-chain alcohols may also be used, for instance ether alcohols or alternatively "Guerbet" alcohols.
Finally, use may also be made of certain more or less long fractions of alcohols of natural origin, for instance coconut ($C_{12}$ to $C_{16}$) or tallow ($C_{16}$ to $C_{18}$) or compounds of diol or cholesterol type.
Use is preferably made of a fatty alcohol comprising from 10 to 24 carbon atoms and more preferentially from 12 to 22 carbon atoms.
As particular examples of fatty alcohols that may preferably be used, mention may be made especially of lauryl alcohol, isostearyl alcohol, oleyl alcohol, 2-butyloctanol, 2-undecylpentadecanol, 2-hexyldecyl alcohol, isocetyl alcohol and octyldodecanol, and mixtures thereof.
According to one advantageous embodiment of the invention, the alcohol is chosen from octyldodecanol;

- <u>optionally hydroxylated monoesters, diesters or triesters of a $C_2$-$C_8$ monocarboxylic or polycarboxylic acid and of a $C_2$-$C_8$ alcohol.</u>
  In particular:

  * optionally hydroxylated monoesters of a $C_2$-$C_8$ carboxylic acid and of a $C_2$-$C_8$ alcohol,
  * optionally hydroxylated diesters of a $C_2$-$C_8$ dicarboxylic acid and of a $C_2$-$C_8$ alcohol, such as diisopropyl adipate, 2-diethylhexyl adipate, dibutyl adipate, diisostearyl adipate or 2-diethylhexyl succinate,

- optionally hydroxylated triesters of a $C_2$-$C_8$ tricarboxylic acid and of a $C_2$-$C_8$ alcohol, such as citric acid esters, such as trioctyl citrate, triethyl citrate, acetyl tributyl citrate or tributyl citrate,

- esters of a $C_2$-$C_8$ polyol and of one or more $C_2$-$C_8$ carboxylic acids, such as glycol diesters of monoacids, such as neopentyl glycol diheptanoate, or glycol or glycerol triesters of monoacids, such as triacetin;

- ester oils, in particular containing at least 18 carbon atoms and even more particularly between 18 and 70 carbon atoms.

Examples that may be mentioned include monoesters, diesters or triesters.
The ester oils may be hydroxylated or non-hydroxylated.
The non-volatile ester oil may for example be chosen from:

* monoesters comprising at least 18 carbon atoms and even more particularly comprising between 18 and 40 carbon atoms in total, in particular the monoesters of formula $R_1COOR_2$ in which $R_1$ represents a saturated or unsaturated, linear or branched or aromatic fatty acid residue comprising from 4 to 40 carbon atoms and $R_2$ represents a hydrocarbon-based chain, which is in particular branched, containing from 4 to 40 carbon atoms, on condition that the sum of the carbon atoms of the radicals $R_1$ and $R_2$ is greater than or equal to 18, for instance Purcellin oil (cetostearyl octanoate), isononyl isononanoate, $C_{12}$ to $C_{15}$ alkyl benzoates, 2-ethylhexyl palmitate, octyldodecyl neopentanoate, 2-octyldodecyl stearate, 2-octyldodecyl erucate, isostearyl isostearate, $C_{12}$-$C_{15}$ alkyl benzoates, such as 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate or 2-octyldodecyl myristate.

Preferably, they are esters of formula $R_1COOR_2$ in which $R_1$ represents a linear or branched, saturated or unsaturated fatty acid residue containing from 4 to 40 carbon atoms and $R_2$ represents a saturated or unsaturated hydrocarbon-based chain that is in particular branched, containing from 4 to 40 carbon atoms, $R_1$ and $R_2$ being such that the sum of the carbon atoms of the radicals $R_1$ and $R_2$ is greater than or equal to 18.
Even more particularly, the ester comprises between 18 and 40 carbon atoms in total.
Preferred monoesters that may be mentioned include isononyl isononanoate, oleyl erucate and/or 2-octyldodecyl neopentanoate;

* monoesters of a fatty acid, in particular containing at least 18 carbon atoms and even more particularly from 18 to 22 carbon atoms, and especially of lanolic acid, oleic acid, lauric acid or stearic acid, and of diols, for instance propylene glycol monoisostearate;
* diesters especially containing at least 18 carbon atoms and even more particularly comprising between 18 and 60 carbon atoms in total and in particular between 18 and 50 carbon atoms in total. Use may thus be made especially of diesters of a dicarboxylic acid and of monoalcohols, preferably such as diisostearyl malate, or glycol diesters of monocarboxylic acids, such as neopentyl glycol diheptanoate, propylene glycol dioctanoate, diethylene glycol diisononanoate or polyglyceryl-2 diisostearate (in particular such as the compound sold under the commercial reference Dermol DGDIS by the company Akzo);
* hydroxylated monoesters and diesters, preferably with a total carbon number of at least 18 carbon atoms and even more particularly ranging from 18 to 70, for instance polyglyceryl-3 diisostearate, isostearyl lactate, octyl hydroxystearate, octyldodecyl hydroxystearate, diisostearyl malate or glyceryl stearate;
* triesters, in particular comprising between 35 and 70 carbon atoms in total, in particular such as triesters of a tricarboxylic acid, such as triisostearyl citrate, or tridecyl trimellitate, or glycol, glycerol or polyglycerol triesters of monocarboxylic acids such as polyglyceryl-2 triisostearate;
* tetraesters, in particular with a total carbon number ranging from 35 to 70, such as pentaerythritol or polyglycerol tetraesters of a monocarboxylic acid, for instance pentaerythrityl tetrapelargonate, pentaerythrityl tetraisostearate, pentaerythrityl tetraisononanoate, glyceryl tris(2-decyl)tetradecanoate, polyglyceryl-2 tetraisostearate or pentaerythrityl tetrakis(2-decyl)tetradecanoate;
* polyesters obtained by condensation of an unsaturated fatty acid dimer and/or trimer and of diol, such as those described in patent application FR 0 853 634, in particular such as dilinoleic acid and 1,4-butanediol. Mention may especially be made in this respect of the polymer sold by Biosynthis under the name Viscoplast 14436H (INCI name: dilinoleic acid/butanediol copolymer), or else copolymers of polyols and of dimer diacids, and esters thereof, such as Hailucent ISDA;
* esters and polyesters of diol dimer and of monocarboxylic or dicarboxylic acid, such as esters of diol dimer and of fatty acid and esters of diol dimer and of dicarboxylic acid dimer, in particular which may be obtained from a

dicarboxylic acid dimer derived in particular from the dimerization of an unsaturated fatty acid especially of $C_8$ to $C_{34}$, especially of $C_{12}$ to $C_{22}$, in particular of $C_{16}$ to $C_{20}$ and more particularly of $C_{18}$, such as esters of dilinoleic diacids and of dilinoleic diol dimers, for instance those sold by the company Nippon Fine Chemical under the trade names Lusplan DD-DA5® and DD-DA7®;

* polyesters resulting from the esterification of at least one triglyceride of hydroxylated carboxylic acid(s) with an aliphatic monocarboxylic acid and with an aliphatic dicarboxylic acid, which is optionally unsaturated, for instance the succinic acid and isostearic acid castor oil sold under the reference Zenigloss by Zenitech;

* hydrocarbon-based plant oils such as fatty acid triglycerides (which are liquid at room temperature), especially of fatty acids containing from 7 to 40 carbon atoms, such as heptanoic or octanoic acid triglycerides or jojoba oil; mention may be made in particular of saturated triglycerides such as caprylic/capric triglyceride and mixtures thereof, for example such as the product sold under the reference Myritol 318 from Cognis, glyceryl triheptanoate, glyceryl trioctanoate, and $C_{18-36}$ acid triglycerides such as those sold under the reference Dub TGI 24 by Stéarineries Dubois, and unsaturated triglycerides such as castor oil, olive oil, ximenia oil or pracaxi oil;

- vinylpyrrolidone/1-hexadecene copolymers, for instance the product sold under the name Antaron V-216 (also known as Ganex V216) by the company ISP (MW = 7300 g/mol);

- $C_{12}$-$C_{26}$ fatty acids, preferably $C_{12}$-$C_{22}$ fatty acids, which are preferably unsaturated, such as oleic acid, linoleic acid or linolenic acid, and mixtures thereof;

- dialkyl carbonates, the 2 alkyl chains possibly being identical or different, such as dicaprylyl carbonate sold under the name Cetiol CC® by Cognis;

- and mixtures thereof.

**Apolar non-volatile hydrocarbon-based oils**

The composition according to the invention can also comprise at least one apolar non-volatile hydrocarbon-based oil.

These oils may be of plant, mineral or synthetic origin.

For the purposes of the present invention, the term "apolar oil" is intended to mean an oil of which the solubility parameter at 25°C, $\delta_a$, is equal to 0 $(J/cm^3)^{½}$.

The definition and calculation of the solubility parameters in the Hansen three-dimensional solubility space are described in the article by C.M. Hansen: The three-dimensional solubility parameters, J. Paint Technol. 39, 105 (1967).

According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{½}$.

The parameters $\delta_p$, $\delta_h$, $\delta_D$ and $\delta_a$ are expressed in $(J/cm^3)^{½}$.

The term "hydrocarbon-based oil" means an oil formed essentially from, or even constituted of, carbon and hydrogen atoms, and optionally oxygen and nitrogen atoms, and not containing any silicon or fluorine atoms. It may contain alcohol, ester, ether, carboxylic acid, amine and/or amide groups. Preferably, the apolar oil consists of carbon and hydrogen atoms, in other words it is free of oxygen or nitrogen atoms.

Preferably, the non-volatile apolar hydrocarbon-based oil may be chosen from linear or branched hydrocarbons of mineral or synthetic origin, such as:

- liquid paraffin or derivatives thereof,
- squalane,
- isoeicosane,
- naphthalene oil,
- polybutenes such as Indopol H-100 (molar mass or MW = 965 g/mol), Indopol H-300 (MW = 1340 g/mol) and Indopol H-1500 (MW = 2160 g/mol) sold or manufactured by the company Amoco,
- polyisobutenes, hydrogenated polyisobutenes such as Parleam® sold by the company Nippon Oil Fats, Panalane

H-300 E sold or manufactured by the company Amoco (MW = 1340 g/mol), Viseal 20000 sold or manufactured by the company Synteal (MW = 6000 g/mol) and Rewopal PIB 1000 sold or manufactured by the company Witco (MW = 1000 g/mol), or alternatively Parleam Lite sold by NOF Corporation,

- decene/butene copolymers, polybutene/polyisobutene copolymers, in particular Indopol L-14,
- polydecenes and hydrogenated polydecenes such as: Puresyn 10 (MW = 723 g/mol) and Puresyn 150 (MW = 9200 g/mol) sold or manufactured by the company Mobil Chemicals, or alternatively Puresyn 6 sold by ExxonMobil Chemical,

  - and mixtures thereof.

**Non-volatile non-phenylated silicone oils**

The term "non-phenyl silicone oil" denotes a silicone oil not bearing any phenyl substituents.
Representative examples of these non-volatile non-phenyl silicone oils which may be mentioned include polydimethylsiloxanes; alkyl dimethicones; vinylmethyl methicones; and also silicones modified with aliphatic groups and/or with functional groups such as hydroxyl, thiol and/or amine groups.
It should be noted that "dimethicone" (INCI name) corresponds to a poly(dimethylsiloxane) (chemical name).
The non-volatile non-phenyl silicone oil is preferably chosen from non-volatile dimethicone oils.
In particular, these oils can be chosen from the following non-volatile oils:

- polydimethylsiloxanes (PDMSs),
- PDMSs comprising aliphatic groups, in particular alkyl or alkoxy groups, which are pendent and/or at the end of the silicone chain, these groups each comprising from 2 to 24 carbon atoms. By way of example, mention may be made of the cetyl dimethicone sold under the commercial reference Abil Wax 9801 from Evonik Goldschmidt,

- PDMSs comprising aliphatic groups, or functional groups such as hydroxyl, thiol and/or amine groups,
- polyalkylmethylsiloxanes substituted with functional groups such as hydroxyl, thiol and/or amine groups,
- polysiloxanes modified with fatty acids, fatty alcohols or polyoxyalkylenes, and mixtures thereof.

Preferably, these non-volatile non-phenylated silicone oils are chosen from polydimethylsiloxanes; alkyl dimethicones and also PDMSs comprising aliphatic groups, in particular $C_2$-$C_{24}$ alkyl groups, and/or functional groups such as hydroxyl, thiol and/or amine groups.
The non-phenylated silicone oil may be chosen in particular from silicones of formula (I):

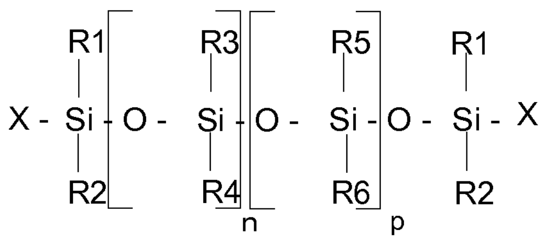

(I)

in which:

$R_1$, $R_2$, $R_5$ and $R_6$ are, together or separately, an alkyl radical containing from 1 to 6 carbon atoms,
$R_3$ and $R_4$ are, together or separately, an alkyl radical containing from 1 to 6 carbon atoms, a vinyl radical, an amine radical or a hydroxyl radical,
X is an alkyl radical containing from 1 to 6 carbon atoms, a hydroxyl radical or an amine radical,
n and p are integers chosen so as to have a fluid compound, in particular of which the viscosity at 25°C is between 9 centistokes (cSt) ($9 \times 10^{-6}$ $m^2/s$) and 800 000 cSt.

As non-volatile non-phenylated silicone oils which can be used according to the invention, mention may be made of those for which:

- the substituents $R_1$ to $R_6$ and X represent a methyl group, and p and n are such that the viscosity is 500 000 cSt,

for example the product sold under the name SE30 by the company General Electric, the product sold under the name AK 500000 by the company Wacker, the product sold under the name Mirasil DM 500 000 by the company Bluestar, and the product sold under the name Dow Corning 200 Fluid 500 000 cSt by the company Dow Corning,

- the substituents $R_1$ to $R_6$ and X represent a methyl group, and p and n are such that the viscosity is 60 000 cSt, for example the product sold under the name Dow Corning 200 Fluid 60 000 CS by the company Dow Corning, and the product sold under the name Wacker Belsil DM 60 000 by the company Wacker,
- the substituents $R_1$ to $R_6$ and X represent a methyl group, and p and n are such that the viscosity is 100 cSt or 350 cSt, for example the products sold respectively under the names Belsil DM100 and Dow Corning 200 Fluid 350 CS by the company Dow Corning,
- the substituents $R_1$ to $R_6$ represent a methyl group, the group X represents a hydroxyl group, and n and p are such that the viscosity is 700 cSt, for example the product sold under the name Baysilone Fluid T0.7 by the company Momentive.

Preferably, if the composition comprises at least one additional non-volatile oil, this oil is chosen from non-volatile polar oils, and in particular ester oils.

More particularly, the composition comprises at least one alcohol ester hydrocarbon-based non-volatile oil.

These oils make it possible to further improve the colour homogeneity of the deposit, while at the same time conserving good properties for the composition during application (for example glidance), without degrading its performance qualities in terms of comfort or persistence of the colour.

The term "alcohol ester hydrocarbon-based oil" means an oil formed essentially from, or even constituted by, carbon, hydrogen, oxygen and optionally nitrogen atoms, and not containing any silicon or fluorine atoms.

In addition, this non-volatile oil comprises at least one ester function and at least one free alcohol function (hydroxyl group).

The alcohol ester non-volatile oil is preferably chosen from the following oils, alone or as mixtures:

* hydroxylated monoesters, diesters and triesters of a $C_2$-$C_8$ monocarboxylic or polycarboxylic acid and of a $C_2$-$C_8$ alcohol, in particular triesters of a hydroxylated $C_2$-$C_8$ tricarboxylic acid and of a $C_2$-$C_8$ alcohol, such as citric acid esters, in particular trioctyl citrate, triethyl citrate or tributyl citrate, and mixtures thereof;
* hydroxylated monoesters, diesters and triesters, preferably with a total carbon number ranging from 18 to 70, more particularly esters of a saturated, unsaturated or aromatic monocarboxylic, dicarboxylic or tricarboxylic acid and of a monoalcohol or polyol, which is preferably saturated, for instance propylene glycol monoisostearate, diisostearyl malate, poly(2-glyceryl) diisostearate (especially such as the compound sold under the trade reference Dermol DGDIS by the company Akzo), poly(3-glyceryl) diisostearate, isostearyl lactate, octyl hydroxystearate, octyldodecyl hydroxystearate, glyceryl stearate, triisostearyl citrate or tridecyl trimellitate, poly(2-glyceryl) triisostearate.

In accordance with an advantageous embodiment of the invention, the alcohol ester oil is not a triglyceride, in other words it is not a triester of glycerol and of a hydroxylated carboxylic acid.

According to this variant, the composition may also comprise one or more hydrocarbon-based non-volatile oils, other than the alcohol ester non-volatile oil(s) described previously.

According to this variant, the composition preferably comprises, besides the abovementioned alcohol ester hydrocarbon-based non-volatile oil and the abovementioned vinyl polymer, at least 15% by weight, relative to the weight of the composition, of at least one phenyl silicone non-volatile oil not comprising any dimethicone fragments and at least one solid dyestuff, preferably at least one pigment.

If the composition comprises one or more additional non-volatile oils, then the content thereof represents from 2% to 20% by weight and preferably from 2% to 15% by weight relative to the total weight of the composition.

According to a very advantageous variant of the invention, the composition comprises a content of hydroxylated ester non-volatile oil(s) ranging from 2% to 20% by weight and preferably from 2% to 15% by weight relative to the total weight of the composition.

## DYESTUFFS

A composition in accordance with the present invention may comprise at least one dyestuff, which may be chosen from water-soluble or water-insoluble, liposoluble or non-liposoluble, organic or mineral dyestuffs, and materials with an optical effect, and mixtures thereof.

For the purposes of the present invention, the term "dyestuff' means a compound that is capable of producing a coloured optical effect when it is formulated in sufficient amount in a suitable cosmetic medium.

The water-soluble dyestuffs used according to the invention are more particularly water-soluble dyes.

For the purposes of the invention, the term "water-soluble dye" means any natural or synthetic, generally organic

compound, which is soluble in an aqueous phase or water-miscible solvents and which is capable of imparting colour. In particular, the term "water-soluble" is intended to mean the capacity of a compound to be dissolved in water, measured at 25°C, to a concentration at least equal to 0.1 g/l (production of a macroscopically isotropic, transparent, coloured or colourless solution). This solubility is in particular greater than or equal to 1 g/l.

As water-soluble dyes that are suitable for use in the invention, mention may be made in particular of synthetic or natural water-soluble dyes, for instance FDC Red 4 (CI: 14700), DC Red 6 (Lithol Rubine Na; CI: 15850), DC Red 22 (CI: 45380), DC Red 28 (CI: 45410 Na salt), DC Red 30 (CI: 73360), DC Red 33 (CI: 17200), DC Orange 4 (CI: 15510), FDC Yellow 5 (CI: 19140), FDC Yellow 6 (CI: 15985), DC Yellow 8 (CI: 45350 Na salt), FDC Green 3 (CI: 42053), DC Green 5 (CI: 61570), FDC Blue 1 (CI: 42090).

As non-limiting illustrations of sources of water-soluble dyestuff(s) that may be used in the context of the present invention, mention may be made in particular of those of natural origin, such as extracts of cochineal carmine, of beetroot, of grape, of carrot, of tomato, of annatto, of paprika, of henna, of caramel and of curcumin.

Thus, the water-soluble dyestuffs that are suitable for use in the invention are especially carminic acid, betanin, anthocyans, enocyanins, lycopene, β-carotene, bixin, norbixin, capsanthin, capsorubin, flavoxanthin, lutein, cryptoxanthin, rubixanthin, violaxanthin, riboflavin, rhodoxanthin, cantaxanthin and chlorophyll, and mixtures thereof.

They may also be copper sulfate, iron sulfate, water-soluble sulfopolyesters, rhodamine, betaine, methylene blue, the disodium salt of tartrazine and the disodium salt of fuchsin.

Some of these water-soluble dyestuffs are in particular permitted for food use. Representatives of these dyes that may be mentioned more particularly include dyes of the carotenoid family, referenced under the food codes E120, E162, E163, E160a-g, E150a, E101, E100, E140 and E141.

According to a particularly preferred embodiment, the water-soluble dyestuff(s) are chosen from the disodium salt of brilliant yellow FCF sold by the company LCW under the name DC Yellow 6, the disodium salt of fuchsin acid D sold by the company LCW under the name DC Red 33, and the trisodium salt of Rouge Allura sold by the company LCW under the name FD & C Red 40.

The term "pigments" should be understood as meaning white or coloured, inorganic (mineral) or organic particles, which are insoluble in the liquid organic phase, and which are intended to colour and/or opacify the composition and/or the deposit produced with the composition.

The pigments may be chosen from mineral pigments, organic pigments and composite pigments (i.e. pigments based on mineral and/or organic materials).

The pigments may be chosen from monochromatic pigments, lakes, nacres, and pigments with an optical effect, for instance reflective pigments and goniochromatic pigments.

The mineral pigments may be chosen from metal oxide pigments, chromium oxides, iron oxides, titanium dioxide, zinc oxides, cerium oxides, zirconium oxides, manganese violet, Prussian blue, ultramarine blue and ferric blue, and mixtures thereof.

Organic lakes are organic pigments formed from a dye attached to a substrate.

The lakes, which are also known as organic pigments, may be chosen from the materials below, and mixtures thereof:

- cochineal carmine;
- organic pigments of azo dyes, anthraquinone dyes, indigoid dyes, xanthene dyes, pyrene dyes, quinoline dyes, triphenylmethane dyes or fluoran dyes. Among the organic pigments that may in particular be mentioned are those known under the following names: D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5, FD&C Yellow No. 6;
- the organic lakes may be insoluble sodium, potassium, calcium, barium, aluminium, zirconium, strontium or titanium salts of acidic dyes such as azo, anthraquinone, indigoid, xanthene, pyrene, quinoline, triphenylmethane or fluoran dyes, these dyes possibly comprising at least one carboxylic or sulfonic acid group.

The organic lakes may also be supported on an organic support such as rosin or aluminium benzoate, for example.

Among the organic lakes, mention may be made in particular of those known under the following names: D&C Red No. 2 Aluminium lake, D&C Red No. 3 Aluminium lake, D&C Red No. 4 Aluminium lake, D&C Red No. 6 Aluminium lake, D&C Red No. 6 Barium lake, D&C Red No. 6 Barium/Strontium lake, D&C Red No. 6 Strontium lake, D&C Red No. 6 Potassium lake, D&C Red No. 7 Aluminium lake, D&C Red No. 7 Barium lake, D&C Red No. 7 Calcium lake, D&C Red No. 7 Calcium/Strontium lake, D&C Red No. 7 Zirconium lake, D&C Red No. 8 Sodium lake, D&C Red No. 9

Aluminium lake, D&C Red No. 9 Barium lake, D&C Red No. 9 Barium/Strontium lake, D&C Red No. 9 Zirconium lake, D&C Red No. 10 Sodium lake, D&C Red No. 19 Aluminium lake, D&C Red No. 19 Barium lake, D&C Red No. 19 Zirconium lake, D&C Red No. 21 Aluminium lake, D&C Red No. 21 Zirconium lake, D&C Red No. 22 Aluminium lake, D&C Red No. 27 Aluminium lake, D&C Red No. 27 Aluminium/Titanium/Zirconium lake, D&C Red No. 27 Barium lake, D&C Red No. 27 Calcium lake, D&C Red No. 27 Zirconium lake, D&C Red No. 28 Aluminium lake, D&C Red No. 30 lake, D&C Red No. 31 Calcium lake, D&C Red No. 33 Aluminium lake, D&C Red No. 34 Calcium lake, D&C Red No. 36 lake, D&C Red No. 40 Aluminium lake, D&C Blue No. 1 Aluminium lake, D&C Green No. 3 Aluminium lake, D&C Orange No. 4 Aluminium lake, D&C Orange No. 5 Aluminium lake, D&C Orange No. 5 Zirconium lake, D&C Orange No. 10 Aluminium lake, D&C Orange No. 17 Barium lake, D&C Yellow No. 5 Aluminium lake, D&C Yellow No. 5 Zirconium lake, D&C Yellow No. 6 Aluminium lake, D&C Yellow No. 7 Zirconium lake, D&C Yellow No. 10 Aluminium lake, FD&C Blue No. 1 Aluminium lake, FD&C Red No. 4 Aluminium lake, FD&C Red No. 40 Aluminium lake, FD&C Yellow No. 5 Aluminium lake, FD&C Yellow No. 6 Aluminium lake.

Mention may also be made of liposoluble dyes, such as, for example, Sudan Red, DC Red 17, DC Green 6, β-carotene, soybean oil, Sudan Brown, DC Yellow 11, DC Violet 2, DC Orange 5 and quinoline yellow.

The chemical substances corresponding to each of the organic dyestuffs cited above are mentioned in the publication "International Cosmetic Ingredient Dictionary and Handbook", 1997 edition, pages 371 to 386 and 524 to 528, published by The Cosmetic, Toiletry and Fragrance Association, the content of which is incorporated into the present patent application by way of reference.

The pigments may also have been subjected to a hydrophobic treatment.

The hydrophobic treatment agent may be chosen from silicones such as methicones, dimethicones and perfluoroalkylsilanes; fatty acids such as stearic acid; metal soaps such as aluminium dimyristate, the aluminium salt of hydrogenated tallow glutamate, perfluoroalkyl phosphates, perfluoroalkylsilanes, perfluoroalkylsilazanes, polyhexafluoropropylene oxides, polyorganosiloxanes comprising perfluoroalkyl perfluoropolyether groups and amino acids; N-acylamino acids or salts thereof; lecithin, isopropyl triisostearyl titanate, and mixtures thereof.

The N-acylamino acids can comprise an acyl group having from 8 to 22 carbon atoms, such as, for example, a 2-ethylhexanoyl, caproyl, lauroyl, myristoyl, palmitoyl, stearoyl or cocoyl group. The salts of these compounds may be aluminium, magnesium, calcium, zirconium, zinc, sodium or potassium salts. The amino acid may be, for example, lysine, glutamic acid or alanine.

The term "alkyl" mentioned in the compounds cited above especially denotes an alkyl group containing from 1 to 30 carbon atoms and preferably containing from 5 to 16 carbon atoms.

Hydrophobically treated pigments are described in particular in patent application EP-A-1 086 683.

For the purposes of the present patent application, the term "nacre" is intended to mean coloured particles of any form, which may or may not be iridescent, in particular produced by certain molluscs in their shell, or alternatively synthesized, and which have a colour effect via optical interference.

Examples of nacres that may be mentioned include nacreous pigments such as titanium mica coated with an iron oxide, mica coated with bismuth oxychloride, titanium mica coated with chromium oxide, titanium mica coated with an organic dye in particular of the abovementioned type, and also nacreous pigments based on bismuth oxychloride. They may also be mica particles, at the surface of which are superposed at least two successive layers of metal oxides and/or of organic dyestuffs.

The nacres may more particularly have a yellow, pink, red, bronze, orangey, brown, gold and/or coppery colour or tint.

As illustrations of nacres that may be introduced as interference pigments into the first composition, mention may be made of the gold-coloured nacres sold in particular by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold in particular by the company Merck under the name Bronze fine (17384) (Colorona) and Bronze (17353) (Colorona) and by the company Engelhard under the name Super bronze (Cloisonne); the orange nacres sold in particular by the company Engelhard under the name Orange 363C (Cloisonne) and Orange MCR 101 (Cosmica) and by the company Merck under the name Passion orange (Colorona) and Matte orange (17449) (Microna); the brown nacres sold in particular by the company Engelhard under the name Nu-antique copper 340XB (Cloisonne) and Brown CL4509 (Chromalite); the nacres with a copper tint sold in particular by the company Engelhard under the name Copper 340A (Timica); the nacres with a red tint sold in particular by the company Merck under the name Sienna fine (17386) (Colorona); the nacres with a yellow tint sold in particular by the company Engelhard under the name Yellow (4502) (Chromalite); the red nacres with a gold tint sold in particular by the company Engelhard under the name Sunstone G012 (Gemtone); the pink nacres sold in particular by the company Engelhard under the name Tan opale G005 (Gemtone); the black nacres with a gold tint sold in particular by the company Engelhard under the name Nu antique bronze 240 AB (Timica), the blue nacres sold in particular by the company Merck under the name Matte blue (17433) (Microna), the white nacres with a silvery tint sold in particular by the company Merck under the name Xirona Silver, and the golden-green pink-orange nacres sold in particular by the company Merck under the name Indian summer (Xirona), and mixtures

thereof.

Advantageously, the content of dyestuff(s) represents from 0.1% to 25% by weight and more specifically from 0.1% to 15% by weight relative to the total weight of the composition.

## FILLERS

The composition according to the invention may optionally comprise at least one or more filler(s) of organic or mineral nature.

The term "filler" should be understood to mean colourless or white solid particles of any shape which are in a form that is insoluble and dispersed in the medium of the composition. These particles, of mineral or organic nature, can give body or rigidity to the composition and/or softness and uniformity to the makeup. The fillers, in particular organic fillers, are not dyestuffs.

The fillers used in the compositions according to the present invention may be in lamellar, globular or spherical form, in the form of fibres or in any other form intermediate between these defined forms.

The fillers according to the invention may or may not be surface-coated, and in particular they may be surface-treated with silicones, amino acids, fluoro derivatives or any other substance that promotes the dispersion and compatibility of the filler in the composition.

Examples of mineral fillers that may be mentioned include talc, mica, silica, hollow silica microspheres, kaolin, calcium carbonate, magnesium carbonate, hydroxyapatite, boron nitride, glass or ceramic microcapsules, or composites of silica and of titanium dioxide, for instance the TSG series sold by Nippon Sheet Glass.

Examples of organic fillers that may be mentioned include polyamide powder (Nylon® Orgasol from Atochem), polyethylene powder, polymethyl methacrylate powder or powders of methyl methacrylate copolymers (for example: Polypore® L 200 - polymethyl methacrylate/ethylene glycol dimethacrylate; Chemdal Corporation), polytetrafluoroethylene powders (for example: Teflon), powders of acrylic acid copolymers (Polytrap from the company Dow Corning), lauroyllysine, hollow polymer microspheres such as those of polyvinylidene chloride/acrylo nitrile (for example: Expancel from Akzo Nobel), synthetic or natural micronized waxes, metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate, silicone fillers, polyurethane powders, and mixtures of these fillers.

As regards the silicone fillers, they may be chosen more particularly from silicone resin microbeads, polymethylsilsesquioxane powders, and powders of crosslinked elastomeric organopolysiloxane coated with silicone resin, and mixtures thereof.

Organopolysiloxane powders coated with silicone resin, for example coated with silsesquioxane resin, as described especially in patent US 5 538 793, are especially suitable for performing the invention. Such elastomeric powders are sold under the names KSP-100, KSP-101, KSP-102, KSP-103, KSP-104 and KSP-105 by the company Shin-Etsu, and have the INCI name: vinyl dimethicone/methicone silsesquioxane crosspolymer. Mention may also be made of powders of crosslinked elastomeric organopolysiloxane coated with silicone resin such as powders of a hybrid silicone functionalized with fluoroalkyl groups, sold in particular under the name KSP-200 by the company Shin-Etsu, or powders of hybrid silicones functionalized with phenyl groups, sold especially under the name KSP-300 by the company Shin-Etsu.

As regards the polymethylsilsesquioxane powders, mention may be made most particularly of silicone resin microbeads, such as those sold under the name Tospearl by the company Momentive Performance Materials, and especially under the reference Tospearl 145 A; and mixtures thereof.

As regards the polyurethane powders, mention may be made in particular of powders of crosslinked polyurethane comprising a copolymer, the said copolymer comprising trimethylol hexyl lactone. It may in particular be a hexamethylene diisocyanate/trimethylol hexyl lactone polymer. Such particles are especially commercially available, for example, under the name Plastic Powder D-400® or Plastic Powder D-800® from the company Toshiki, and mixtures thereof.

Preferably, the organic filler is chosen from silicone fillers or mixtures thereof, in particular from powders of crosslinked elastomeric organopolysiloxane coated with silicone resin.

A composition used according to the invention may comprise one or more fillers advantageously in a content ranging from 0.1% to 15% by weight and in particular from 1% to 10% by weight relative to the total weight of the composition.

In accordance with one variant of the invention, the composition comprises at least one mineral filler, advantageously in a content of between 0.1% to 15% by weight and in particular from 1% to 10% by weight relative to the total weight of the composition.

According to another variant, the composition comprises at least one organic filler, advantageously in a content of less than 8% by weight relative to the weight of the composition, and more particularly in a maximum content of 6% by weight relative to the weight of the composition. In accordance with an even more preferential variant, and if the composition comprises any, the content of organic filler is between 0.1% and 6% by weight relative to the weight of the

composition.

In accordance with this variant, the composition preferably comprises at least 10% by weight of one or more phenyl silicone non-volatile oils not containing any dimethicone fragments.

The presence of these fillers makes it possible to further reduce the phenomenon of migration of the composition.

## ADDITIVES

The composition according to the invention may furthermore comprise any of the ingredients conventionally used as additives in the cosmetics and dermatology field.

These additives are advantageously chosen from surfactants, antioxidants, thickeners, sweeteners, basifying or acidifying preserving agents, and mixtures thereof, and may be chosen advantageously from those proposed in Table 1 of the Codex Alimentarius.

As antioxidant, a composition in accordance with the invention may advantageously comprise at least one pentaerythrityl di-t-butyl hydroxycinnamate.

A composition according to the invention may also contain flavourings and/or fragrances.

As cosmetic active agents that may be used in the invention, mention may be made of sunscreens, vitamins A, E, C and B3, provitamins such as D-panthenol, calmatives such as $\alpha$-bisabolol, *Aloe vera,* allantoin, plant extracts or essential oils, protective or restructuring agents, refreshing agents such as menthol and derivatives thereof, emollients, moisturizers and essential fatty acids, and mixtures thereof.

The amounts of each of these various ingredients, if they are present, are those conventionally used in the fields under consideration, and range, for example, from 0.01% to 10% by weight relative to the total weight of the composition.

Needless to say, those skilled in the art will take care to select this or these optional additional compound(s), and/or the amount thereof, such that the advantageous properties of the composition according to the invention are not, or are not substantially, adversely affected by the envisaged addition.

The example hereinafter is given as a non-limiting illustration of the field of the invention.

## EXAMPLES

The solid lipstick composition was prepared, the ingredients and proportions of which are collated in the table below (the percentages are expressed as weight percentages of raw material):

| | Invention | comparative |
|---|---|---|
| Phenyl trimethicone (KF 56 A from Shin-Etsu) | 44.98 | 44.98 |
| $C_{12}$-$C_{15}$ Alkyl benzoate | 7.40 | 7.40 |
| Polyphenyltrimethylsiloxydimethylsiloxane (Belsil PDM 1000 from Wacker) | 7.54 | 7.54 |
| Butyl acrylate copolymer containing dendritic silicone side chains: Tris((trimethylsiloxy) siloxyethyldimethylsiloxy)silylpropy 1 methacrylate in isododecane (40/60) sold under the reference Dow Corning FA 4002 ID by Dow Corning | 21 | 21 |
| Linear long chain ($C_{30}$-$C_{50}$) fatty alcohol (Performacol 550 Alcohol from New Phase Technologies) | 8.8 | |
| C30-45 alkyldimethylsilyl polypropylsilsesquioxane (DOW CORNING SW-8005 C30 RESIN WAX de Dow Corning) | - | 8.8 |
| Candelilla wax | 0.2 | 0.2 |
| Isohexadecane | 2.00 | 2.00 |
| Pigments | 8.00 | 8.00 |
| Fragrance | 0.08 | 0.08 |

The pigments are ground in part of the oily phase.

The remaining oily phase, the waxes, the isohexadecane and the acrylate polymer from Dow Corning are then placed in a heating pan with moderate stirring at 100°C.

Stirring is continued until the mixture is homogeneous.

The composition is then poured into moulds and left to cool.
A stick of suitable hardness is obtained in the case of the composition according to the invention whereas in the case of the comparative experiment, the composition is very fluid when poured into moulds and yields a very soft stick.
The wand of lipstick obtained according to the invention is glidant on application, depositing a fine, comfortable, non-tacky film with good persistence.

## Claims

1. Anhydrous solid cosmetic composition comprising:

   a) at least one vinyl polymer comprising at least one carbosiloxane dendrimer-based unit;
   b) at least two hydrocarbon-based volatile oils,
   c) at least one saturated or unsaturated, preferably linear, solid fatty alcohol whose melting point is greater than or equal to 40°C, comprising from 16 to 60 carbon atoms representing from 5% to 20% by weight, relative to the total weight of the composition.

2. Composition according to the preceding claim, **characterized in that** the vinyl polymer comprising at least one carbosiloxane dendrimer-based unit has a molecular side chain containing a carbosiloxane dendrimer structure, and is the product of polymerization of:

   (A) from 0 to 99.9 parts by weight of a vinyl monomer; and
   (B) from 100 to 0.1 parts by weight of a carbosiloxane dendrimer of formula (I) below:

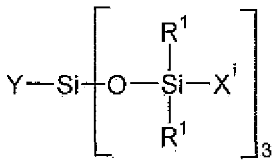
(I)

in which:

- $R^1$ represents an aryl group containing from 5 to 10 carbon atoms or an alkyl group containing from 1 to 10 carbon atoms;
- $X^i$ represents a silylalkyl group which, when i = 1, is represented by formula (II):

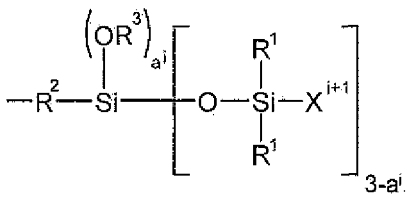
(II)

in which:

- $R^1$ is as defined above in formula (I),
- $R^2$ represents an alkylene radical containing from 2 to 10 carbon atoms,
- $R^3$ represents an alkyl group containing from 1 to 10 carbon atoms,
- $X^{i+i}$ is chosen from: a hydrogen atom, an alkyl group containing from 1 to 10 carbon atoms, an aryl group containing from 5 to 10 carbon atoms and a silylalkyl group defined above of formula (II) with i = i + 1,

. i is an integer from 1 to 10 which represents the generation of said silylalkyl group, and
.$a^i$ is an integer from 0 to 3;

- Y represents a radical-polymerizable organic group chosen from:

  - organic groups containing a methacrylic group or an acrylic group, said organic groups being represented by the formulae:

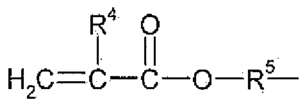
or
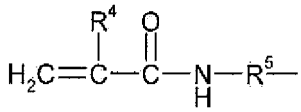

in which:

  * $R^4$ represents a hydrogen atom or an alkyl group containing from 1 to 10 carbon atoms; and
  * $R^5$ represents an alkylene group containing from 1 to 10 carbon atoms; and

- organic groups containing a styryl group of formula:

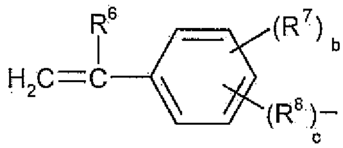

in which:

  * $R^6$ represents a hydrogen atom or an alkyl group containing from 1 to 10 carbon atoms;
  * $R^7$ represents an alkyl group containing from 1 to 10 carbon atoms;
  * $R^8$ represents an alkylene group containing from 1 to 10 carbon atoms;
  * b is an integer from 0 to 4; and
  * c is 0 or 1, such that, if c is 0, $-(R^8)_c-$ represents a bond.

**3.** Composition according to the preceding claim, in which the carbosiloxane dendrimer is represented by the following formula:

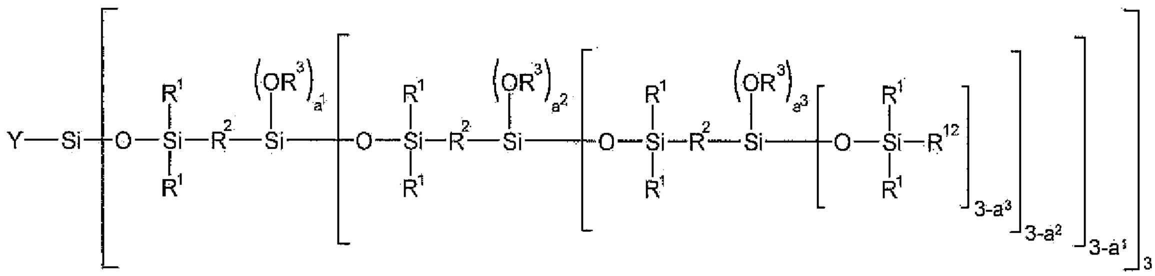

in which:

- Y, $R^1$, $R^2$ and $R^3$ are as defined in Claim 2;

  . $a^1$, $a^2$ and $a^3$ correspond to the definition of $a^i$ according to Claim 2; and

- $R^{12}$ is H, an aryl group containing from 5 to 10 carbon atoms or an alkyl group containing from 1 to 10 carbon atoms.

**4.** Composition according to either of Claims 2 and 3, in which the carbosiloxane dendrimer is represented by one of the following formulae:

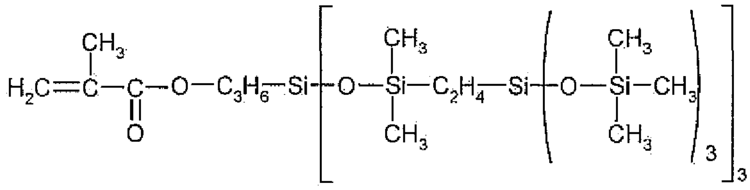

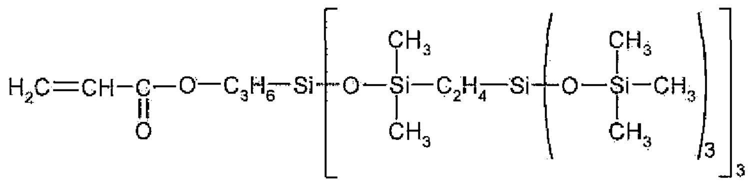

5. Composition according to any one of the preceding claims, **characterized in that** the content of vinyl polymer(s) represents from 0.5% to 20% and in particular from 1% to 15% relative to the weight of the composition.

6. Composition according to any one of the preceding claims, **characterized in that** the hydrocarbon-based volatile oils are apolar, comprise from 8 to 16 carbon atoms and are linear or branched alkanes.

7. Composition according to the preceding claim, **characterized in that** the content of volatile oil(s) represents from 0.5% to 40% by weight and especially from 1% to 30% by weight, relative to the total weight of the said composition.

8. Composition according to any one of the preceding claims, **characterized in that** the composition comprises at least one saturated or unsaturated, linear or branched, preferably linear, solid fatty alcohol, whose melting point is greater than or equal to 40°C, comprising from 18 to 60 carbon atoms.

9. Composition according to the preceding claim, **characterized in that** the content of solid fatty alcohol(s) whose melting point is greater than or equal to 40°C represents from 5% to 15% by weight and even more particularly from 7% to 15% by weight, relative to the total weight of the composition.

10. Composition according to any one of the preceding claims, **characterized in that** the composition comprises at least one alcohol or alcohol-derived additive chosen from:

- solid fatty alcohols with a melting point between 25°C and less than 40°C,
- linear or branched, saturated or unsaturated, monooxyalkylenated or polyoxyalkylenated $C_2$-$C_3$ fatty alcohols containing at least 14 carbon atoms, which are solid at 25°C,
- liposoluble polyethers resulting from polyetherification between one or more $C_2$-$C_{100}$ and preferably $C_2$-$C_{50}$ diols,
- or mixtures thereof.

11. Composition according to the preceding claim, **characterized in that** the content of alcohol or alcohol-derived additive(s) is between 1% and 10% by weight and preferably between 3% and 7.5% by weight, relative to the total weight of the composition.

12. Composition according to any one of the preceding claims, **characterized in that** it comprises at least one wax, other than the solid fatty alcohols whose melting point is greater than or equal to 40°C, and other than the alcohol or alcohol-derived additives, more particularly chosen from polar or apolar hydrocarbon-based waxes, or mixtures thereof.

13. Composition according to the preceding claim, **characterized in that** when the composition comprises any, the content of apolar wax represents between 0.1% and 5% by weight relative to the composition, and when the composition comprises any, the content of polar wax represents between 0.1% and 5% by weight relative to the composition.

14. Composition according to any one of the preceding claims, **characterized in that** it comprises at least one phenyl silicone non-volatile oil not comprising any dimethicone fragments, preferably in a content of between 10% and 50%

by weight and preferably between 15% and 40% by weight relative to the weight of the composition.

**15.** Composition according to any one of the preceding claims, **characterized in that** the composition comprises one or more additional non-volatile oils chosen from polar or apolar hydrocarbon-based non-volatile oils or from non-phenyl silicone non-volatile oils, and also mixtures thereof.

**16.** Composition according to the preceding claim, **characterized in that** the content of additional non-volatile oil(s) is between 2% and 20% by weight and preferably from 2% to 15% by weight, relative to the weight of the composition.

**17.** Composition according to any one of the preceding claims, **characterized in that** the composition comprises at least one dyestuff, chosen in particular from water-soluble or water-insoluble, liposoluble or non-liposoluble, organic or mineral dyestuffs, and materials with an optical effect, and mixtures thereof.

**18.** Process for making up and/or caring for the lips, in which the composition according to any one of the preceding claims is applied to the lips.

## Patentansprüche

**1.** Wasserfreie feste kosmetische Zusammensetzung, umfassend:

a) wenigstens ein Vinylpolymer, das wenigstens eine Einheit auf Carbosiloxan-Dendrimer-Basis umfasst;
b) wenigstens zwei flüchtige Öle auf Kohlenwasserstoffbasis;
c) wenigstens einen gesättigten oder ungesättigten, vorzugsweise linearen, festen Fettalkohol, dessen Schmelzpunkt größer als oder gleich 40 °C ist, umfassend von 16 bis 60 Kohlenstoffatome, der von 5 Gew.-% bis 20 Gew.-% bezogen auf das Gesamtgewicht der Zusammensetzung darstellt.

**2.** Zusammensetzung gemäß dem vorstehenden Anspruch, **dadurch gekennzeichnet, dass** das Vinylpolymer, das wenigstens eine Einheit auf Carbosiloxan-Dendrimer-Basis umfasst, eine molekulare Seitenkette aufweist, die eine Carbosiloxan-Dendrimer-Struktur aufweist, und das Polymerisationsprodukt ist von:

(A) von 0 bis 99,9 Gewichtsteilen an einem Vinylmonomer; und
(B) von 100 bis 0,1 Gewichtsteilen an einem Carbosiloxan-Dendrimer der nachstehenden Formel (I):

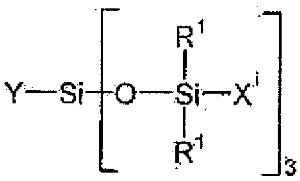

(I)

wobei:

- $R^1$ eine Arylgruppe, die von 5 bis 10 Kohlenstoffatome enthält, oder eine Alkylgruppe, die von 1 bis 10 Kohlenstoffatome enthält, darstellt;
- $X^i$ eine Silylalkylgruppe darstellt, die, wenn i = 1, durch die Formel (II) dargestellt wird:

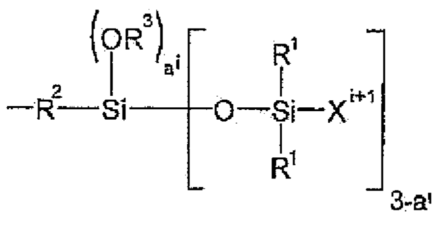

(II)

wobei:

• $R^1$ wie vorstehend in Formel (I) definiert ist,
• $R^2$ einen Alkylenrest, der von 2 bis 10 Kohlenstoffatome enthält, darstellt,
• $R^3$ eine Alkylgruppe, die von 1 bis 10 Kohlenstoffatome enthält, darstellt,
• $X^{i+1}$ ausgewählt ist aus: einem Wasserstoffatom, einer Alkylgruppe, die von 1 bis 10 Kohlenstoffatome enthält, einer Arylgruppe, die von 5 bis 10 Kohlenstoffatome enthält, und einer wie vorstehend definierten Silylalkylgruppe der Formel (II) mit i = i + 1,
• i eine ganze Zahl von 1 bis 10 ist, die die Generation der Silylalkylgruppe darstellt und
• $a^i$ eine ganze Zahl von 0 bis 3 ist;

- Y eine radikalpolymerisierbare organische Gruppe darstellt, ausgewählt aus:

• organischen Gruppen, die eine Methacrylgruppe oder eine Acrylgruppe enthalten, wobei die organischen Gruppen durch die Formeln:

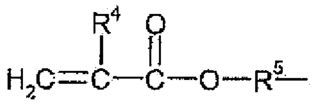

oder

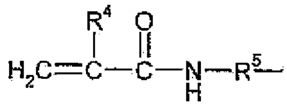

,

dargestellt werden, wobei:

* $R^4$ ein Wasserstoffatom oder eine Alkylgruppe, die von 1 bis 10 Kohlenstoffatome enthält, darstellt; und
* $R^5$ eine Alkylengruppe, die von 1 bis 10 Kohlenstoffatome enthält, darstellt; und

• organischen Gruppen, die eine Styrylgruppe der Formel:

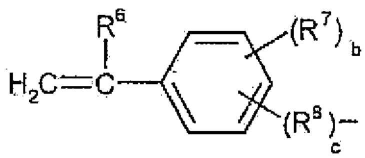

enthalten, wobei:

* $R^6$ ein Wasserstoffatom oder eine Alkylgruppe, die von 1 bis 10 Kohlenstoffatome enthält, darstellt;
* $R^7$ eine Alkylgruppe, die von 1 bis 10 Kohlenstoffatome enthält, darstellt;
* $R^8$ eine Alkylengruppe, die von 1 bis 10 Kohlenstoffatome enthält, darstellt;
* b eine ganze zahl von 0 bis 4 ist; und
* c 0 oder 1 ist, so dass, wenn c 0 ist, $-(R^8)_c-$ eine Bindung darstellt.

**3.** Zusammensetzung gemäß dem vorstehenden Anspruch, wobei das Carbosiloxan-Dendrimer durch die folgende Formel dargestellt wird:

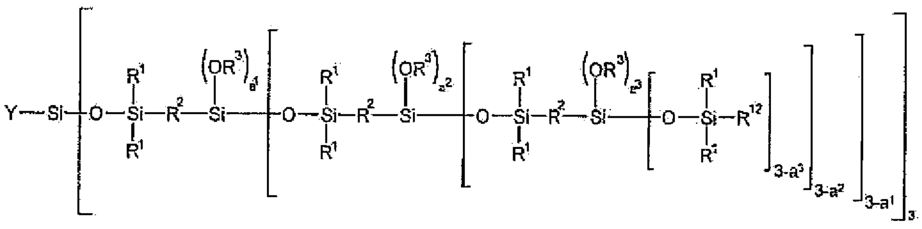

wobei:

• Y, $R^1$, $R^2$ und $R^3$ wie in Anspruch 2 definiert sind;
• $a^1$, $a^2$ und $a^3$ der Definition von $a^i$ gemäß Anspruch 2 entsprechen; und

• $R^{12}$ H, eine Arylgruppe, die von 5 bis 10 Kohlenstoffatome enthält, oder eine Alkylgruppe, die von 1 bis 10 Kohlenstoffatome enthält, ist.

**4.** Zusammensetzung gemäß dem Ansprüche 2 und 3, wobei das Carbosiloxan-Dendrimer durch eine der folgenden, Formeln dargestellt wird:

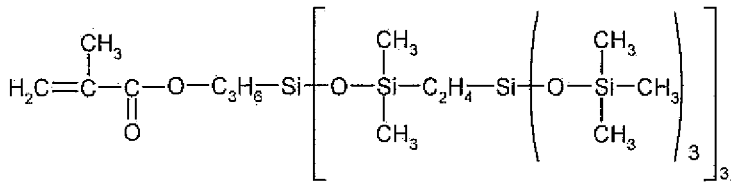

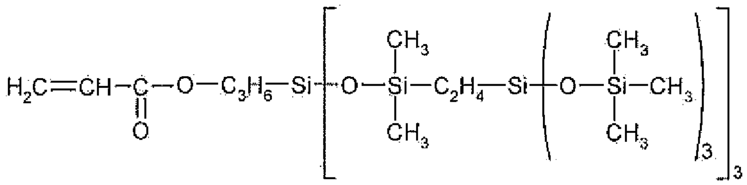

**5.** Zusammensetzung gemäß einem der vorstehenden Ansprüche, **dadurch gekennzeichnet, dass** der Gehalt an Vinylpolymer(en) von 0,5 % bis 20 %, insbesondere von 1 % bis 15 %, bezogen auf das Gewicht der Zusammensetzung darstellt.

**6.** Zusammensetzung gemäß einem der vorstehenden Ansprüche, **dadurch gekennzeichnet, dass** die flüchtigen Öle auf Kohlenwasserstoffbasis apolar sind, von 8 bis 16 Kohlenstoffatome umfassen und lineare oder verzweigte Alkane sind.

**7.** Zusammensetzung gemäß dem vorstehenden Anspruch, **dadurch gekennzeichnet, dass** der Gehalt an flüchtigen Öl(en) von 0,5 Gew.-% bis 40 Gew.-%, insbesondere von 1 Gew.-% bis 30 Gew.-%, bezogen auf das Gesamtgewicht der Zusammensetzung darstellt.

**8.** Zusammensetzung gemäß einem der vorstehenden Ansprüche, **dadurch gekennzeichnet, dass** die Zusammensetzung wenigstens einen gesättigten oder ungesättigten, linearen oder verzweigten, vorzugsweise linearen, festen Fettalkohol umfasst, dessen Schmelzpunkt größer als oder gleich 40 °C ist, umfassend 18 bis 60 Kohlenstoffatome.

**9.** Zusammensetzung gemäß dem vorstehenden Anspruch, **dadurch gekennzeichnet, dass** der Gehalt an festen Fettalkohol(en), deren Schmelzpunkt größer als oder gleich 40 °C ist, von 5 Gew.-% bis 15 Gew.-%, insbesondere von 7 Gew.-% bis 15 Gew.-%, bezogen auf das Gesamtgewicht der Zusammensetzung darstellt.

**10.** Zusammensetzung gemäß einem der vorstehenden Ansprüche, **dadurch gekennzeichnet, dass** die Zusammensetzung wenigstens einen Alkohol oder Alkohol-abgeleiteten Zusatzstoff umfasst, ausgewählt aus:

• festen Fettalkoholen mit einem Schmelzpunkt zwischen 25 °C und weniger als 40 °C,
• linearen öder verzweigten, gesättigten oder ungesättigten, monooxyalkylenierten oder polyoxyalkylenierten $C_2$-$C_3$Fettalkoholen, die wenigstens 14 Kohlenstoffatome enthalten und die bei 25 °C fest sind,
• fettlöslichen Polyethern, die durch Polyverethern zwischen einem oder mehreren $C_2$-$C_{100}$-, vorzugsweise $C_2$-$C_{50}$Diolen, erhalten sind,
• und Gemischen davon.

**11.** Zusammensetzung gemäß dem vorstehenden Anspruch, **dadurch gekennzeichnet, dass** der Gehalt an Alkohol oder Alkohol-abgeleiteten Zusatzstoff(en) zwischen 1 Gew.-% und 10 Gew.-%, vorzugsweise zwischen 3 Gew.-% und 7,5 Gew.-%, bezogen auf das Gesamtgewicht der Zusammensetzung beträgt.

**12.** Zusammensetzung gemäß einem der vorstehenden Ansprüche, **dadurch gekennzeichnet, dass** sie wenigstens ein Wachs, das von den festen Fettalkoholen, deren Schmelzpunkt größer oder gleich 40 °C ist, verschieden ist, und von dem Alkohol oder den Alkohol-abgeleiteten Zusatzstoffen verschieden ist, insbesondere ausgewählt aus polaren und apolaren Wachsen auf Kohlenwasserstoffbasis und Gemischen davon umfasst.

**13.** Zusammensetzung gemäß dem vorstehenden Anspruch, **dadurch gekennzeichnet, dass**, falls die Zusammensetzung es umfasst, der Gehalt an polarem Wachs zwischen 0,1 Gew.-% und 5 Gew.-% bezogen auf die Zusammensetzung darstellt, und, falls die Zusammensetzung es umfasst, der Gehalt an polarem Wachs zwischen 0,1 Gew.-% und 5 Gew.-% bezogen auf die Zusammensetzung darstellt.

**14.** Zusammensetzung gemäß einem der vorstehenden Ansprüche, **dadurch gekennzeichnet, dass** sie wenigstens ein nichtflüchtiges Phenylsiliconöl umfasst, das keine Dimethiconfragmente umfasst, vorzugsweise in einem Gehalt von zwischen 10 Gew.-% und 50 Gew.-%, vorzugsweise zwischen 15 Gew.-% und 40 Gew.-%, bezogen auf das Gewicht der Zusammensetzung.

**15.** Zusammensetzung gemäß einem der vorstehenden Ansprüche, **dadurch gekennzeichnet, dass** die Zusammensetzung ein oder mehrere zusätzliche nichtflüchtige Öle ausgewählt aus polaren und apolaren nichtflüchtigen Ölen auf Kohlenwasserstoffbasis oder aus nichtflüchtigen Nichtphenyl-Siliconölen und Gemischen davon umfasst.

**16.** Zusammensetzung gemäß dem vorstehenden Anspruch, **dadurch gekennzeichnet, dass** der Gehalt an zusätzlichen nichtflüchtigen Öl(en) zwischen 2 Gew.-% und 20 Gew.-%, vorzugsweise von 2 Gew.-% bis 15 Gew.-%, bezogen auf das Gewicht der Zusammensetzung beträgt.

**17.** Zusammensetzung gemäß einem der vorstehenden Ansprüche, **dadurch gekennzeichnet, dass** die Zusammensetzung wenigstens einen Farbstoff umfasst, insbesondere ausgewählt aus wasserlöslichen oder wasserunlöslichen, fettlöslichen oder nicht fettlöslichen, organischen oder mineralischen Farbstoffen, und Materialien mit einem optischen Effekt und Gemischen davon.

**18.** Verfahren zum Make-up und/oder zur Pflege der Lippen, wobei die Zusammensetzung gemäß einem der vorstehenden Ansprüche auf die Lippen aufgebracht wird.

**Revendications**

**1.** Composition cosmétique solide anhydre, comprenant :

a) au moins un polymère vinylique comprenant au moins un motif dérivé de dendrimère carbosiloxane ;
b) au moins deux huiles volatiles hydrocarbonées,
c) au moins un alcool gras solide dont la température de fusion est supérieure ou égale à 40°C, saturé ou insaturé, de préférence linéaire, comprenant de 16 à 60 atomes de carbone, représentant de 5 % à 20 % en poids, par rapport au poids total de la composition.

**2.** Composition selon la revendication précédente, **caractérisée en ce que** le polymère vinylique comprenant au moins un motif dérivé de dendrimère carbosiloxane possède une chaîne moléculaire latérale contenant une structure de dendrimère carbosiloxane et est issu de la polymérisation de :

(A) de 0 à 99,9 parties en poids d'un monomère vinylique ; et
(B) de 100 à 0,1 parties en poids d'un dendrimère carbosiloxane de formule (I) suivante :

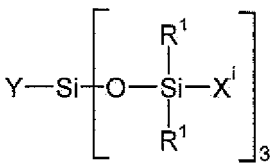

(I)

dans laquelle :

- $R^1$ représente un groupe aryle de 5 à 10 atomes de carbone ou un groupe alkyle de 1 à 10 atomes de carbone ;
- $X^i$ représente un groupe silylalkyle qui, lorsque i = 1, est représenté par la formule (II) :

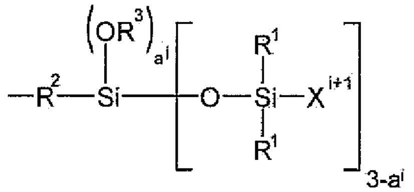

(II)

dans laquelle :

. $R^1$ est tel que défini ci-dessus dans la formule (I),
. $R^2$ représente un radical alkylène de 2 à 10 atomes de carbone,
. $R^3$ représente un groupe alkyle de 1 à 10 atomes de carbone,
. $X^{i+i}$ est choisi parmi : un atome d'hydrogène, un groupe alkyle de 1 à 10 atomes de carbone, un groupe aryle de 5 à 10 atomes de carbone et un groupe silylalkyle défini ci-dessus de formule (II) avec i = i + 1,
. i est un nombre entier de 1 à 10 qui représente la génération dudit groupe silylalkyle, et
. $a^i$ est un nombre entier de 0 à 3 ;

- Y représente un groupe organique polymérisable à l'aide de radicaux choisi parmi :

. des groupes organiques contenant un groupe méthacrylique ou un groupe acrylique, lesdits groupes organiques étant représentés par les formules :

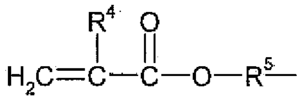

ou

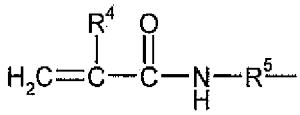

dans lesquelles :

* $R^4$ représente un atome d'hydrogène ou un groupe alkyle de 1 à 10 atomes de carbone ; et
* $R^5$ représente un groupe alkylène de 1 à 10 atomes de carbone ; et

. des groupes organiques contenant un groupe styryle de formule :

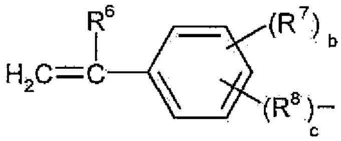

dans laquelle :

* $R^6$ représente un atome d'hydrogène ou un groupe alkyle de 1 à 10 atomes de carbone ;
* $R^7$ représente un groupe alkyle de 1 à 10 atomes de carbone ;
* $R^8$ représente un groupe alkylène de 1 à 10 atomes de carbone ;
* b est un nombre entier de 0 à 4 ; et
* c vaut 0 ou 1, de sorte que, si c vaut 0, $-(R^8)_c-$représente une liaison.

**3.** Composition selon la revendication précédente, dans laquelle le dendrimère carbosiloxane est représenté par la formule suivante :

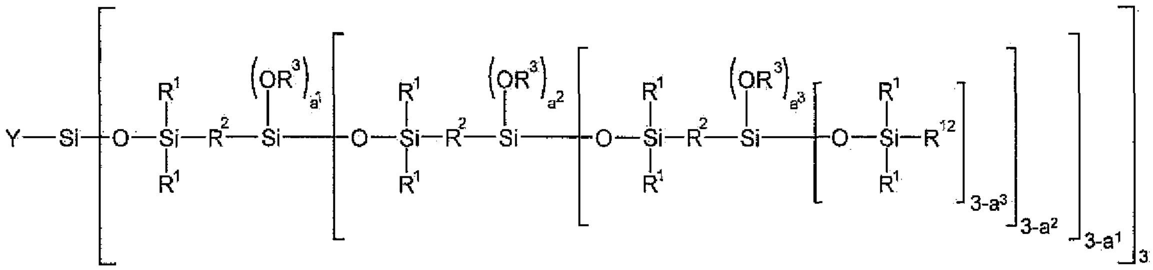

dans laquelle :

. Y, $R^1$, $R^2$ et $R^3$ sont tels que définis dans la revendication 2 ;
. $a^1$, $a^2$ et $a^3$ répondent à la définition de $a^i$ selon la revendication 2 ; et
. $R^{12}$ est H, un groupe aryle de 5 à 10 atomes de carbone ou un groupe alkyle de 1 à 10 atomes de carbone.

**4.** Composition selon l'une quelconque des revendications 2 et 3, dans laquelle le dendrimère carbosiloxane est représenté par l'une des formules suivantes :

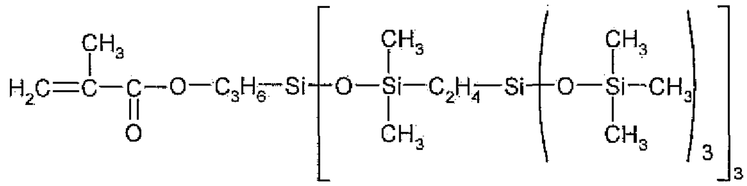

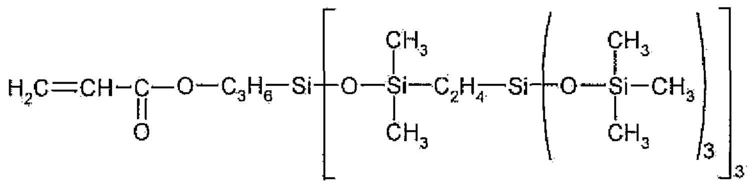

**5.** Composition selon l'une quelconque des revendications précédentes, **caractérisée en ce que** la teneur en polymère(s) vinylique(s) représente de 0,5 % à 20 %, en particulier de 1 % à 15 %, par rapport au poids de la composition.

**6.** Composition selon l'une quelconque des revendications précédentes, **caractérisée en ce que** les huiles volatiles hydrocarbonées sont apolaires, comprennent de 8 à 16 atomes de carbone, et sont des alcanes linéaires ou ramifiés.

**7.** Composition selon la revendication précédente, **caractérisée en ce que** la teneur en huile(s) volatile(s) représente 0,5 % à 40 % en poids, notamment de 1 % à 30 % en poids, par rapport au poids total de ladite composition.

**8.** Composition selon l'une quelconque des revendications précédentes, **caractérisée en ce que** la composition comprend au moins au moins un alcool gras solide, saturé ou insaturé, linéaire ou ramifié, de préférence linéaire, dont la température de fusion est supérieure ou égale à 40°C, comprenant de 18 à 60 atomes de carbone.

**9.** Composition selon la revendication précédente, **caractérisée en ce que** la teneur en alcool (s) gras solide(s) dont la température de fusion est supérieure ou égale à 40°C, représente de 5 % à 15 % en poids, encore plus particulièrement de 7 % à 15 % en poids, par rapport au poids total de la composition.

**10.** Composition selon l'une quelconque des revendications précédentes, **caractérisée en ce que** la composition comprend au moins un additif alcool ou dérivé d'alcool choisi parmi :

• les alcools gras solides dont la température de fusion est comprise entre 25°C et moins de 40°C,
• les alcools gras ayant au moins 14 atomes de carbone, linéaires ou ramifiés, saturés ou insaturés, monooxyalkylénés ou poly-oxyalkylénés en $C_2$-$C_3$, solides à 25°C,
• les polyéthers liposolubles résultant de la polyéthérification entre un ou plusieurs diols en $C_2$-$C_{100}$, de préfé-

rence en $C_2$-$C_{50}$,
• ou leurs mélanges.

**11.** Composition selon la revendication précédente, **caractérisée en ce que** la teneur en additif(s) alcool ou dérivé(s) d'alcool est comprise entre 1 % et 10 % en poids, de préférence entre 3 % et 7,5 % en poids, par rapport au poids total de la composition.

**12.** Composition selon l'une quelconque des revendications précédentes, **caractérisée en ce qu'**elle comprend au moins une cire, différente des alcools gras solides dont la température de fusion est supérieure ou égale à 40°C, et différente des additifs alcool ou dérivés d'alcool, plus particulièrement, choisie parmi les cires hydrocarbonées polaires ou apolaires, ou leurs mélanges.

**13.** Composition selon la revendication précédente, **caractérisée en ce que**, lorsque la composition en comprend, la teneur en cire apolaire représente entre 0,1 % et 5 % en poids par rapport à la composition ; et lorsque la composition en comprend, la teneur en cire polaire représente entre 0,1 % et 5 % en poids par rapport à la composition.

**14.** Composition selon l'une quelconque des revendications précédentes, **caractérisée en ce qu'**elle comprend au moins une huile non volatile siliconée phénylée, ne comprenant pas de fragment diméthicone, de préférence dans une teneur comprise entre 10 % et 50 % en poids, de préférence entre 15 % et 40 % en poids, par rapport au poids de la composition.

**15.** Composition selon l'une quelconque des revendications précédentes, **caractérisée en ce que** la composition comprend une ou plusieurs huiles non volatiles additionnelles choisies parmi les huiles non volatiles hydrocarbonées, polaires ou apolaires ou parmi les huiles non volatiles siliconées non phénylées, ainsi que leurs mélanges.

**16.** Composition selon la revendication précédente, **caractérisée en ce que** la teneur en huile(s) non volatile(s) additionnelle(s) est comprise entre 2 et 20 % en poids, de préférence de 2 % à 15 % en poids, par rapport au poids de la composition.

**17.** Composition selon l'une quelconque des revendications précédentes, **caractérisée en ce que** la composition comprend au moins une matière colorante, en particulier choisie parmi les matières colorantes hydrosolubles ou non, liposolubles ou non, organiques ou inorganiques, les matériaux à effet optique, et leurs mélanges.

**18.** Procédé de maquillage et/ou de soin des lèvres dans lequel la composition selon l'une quelconque des revendications précédentes est appliquée sur les lèvres.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 9171154 A
- JP HEI9171154 B
- EP 0963751 A
- EP 1055674 A
- WO 03045337 A
- WO 2008155059 A
- FR 2792190 A
- FR 0853634
- EP 1086683 A
- US 5538793 A

### Non-patent literature cited in the description

- Long lasting and meal resistance in lipsticks and lipcreams using silicone acrylate copolymers. RESEARCH DISCLOSURE. MASON PUBLICATIONS, 01 May 2006, vol. 505
- Silicone acrylate copolymers in lipstick and color cosmetics. RESEARCH DISCLOSURE. MASON PUBLICATIONS, 01 July 2005, vol. 495
- **C.M. HANSEN.** The three-dimensional solubility parameters. *J. Paint Technol.,* 1967, vol. 39, 105
- International Cosmetic Ingredient Dictionary and Handbook. The Cosmetic, Toiletry and Fragrance Association, 1997, 371-386, 524-528